# EUROPEAN PATENT SPECIFICATION

(11) **EP 4 216 801 B1**
(45) Date of publication and mention of the grant of the patent: **13.08.2025**
(21) Application number: 20954481.6
(22) Date of filing: 24.09.2020
(51) Int. Cl.: A61B 5/00, G16H 15/00, G16H 20/60, G16H 40/63, G16H 40/67, G16H 50/20, G16H 50/70

(54) **HYDRATION TRACKING USING BASELINE CALIBRATION**
HYDRIERUNGSVERFOLGUNG UNTER VERWENDUNG VON GRUNDLINIENKALIBRIERUNG
SUIVI D'HYDRATATION À L'AIDE D'UN ÉTALONNAGE DE LIGNE DE BASE

(43) Date of publication of application: 02.08.2023
(73) Proprietor: QUALCOMM Incorporated, San Diego, CA 92121-1714 (US)
(72) Inventor: CHEN, Wen, San Diego, CA 92121-1714 (US); GUEST, Daniel James, San Diego, CA 92121-1714 (US); RAMP, Katheryn Victoria, San Diego, CA 92121-1714 (US); DAI, Yujie, San Diego, CA 92121-1714 (US)
(74) Representative: Reddie & Grose LLP
(86) International application number: PCT/CN2020/117314
(87) International publication number: WO 2022/061629

(56) References cited:
- WO-A1-2015/200904
- WO-A2-2008/132636
- CN-A- 106 132 289
- US-A1- 2011 125 063
- US-A1- 2015 126 824
- US-A1- 2016 026 767
- US-A1- 2020 135 319

## Description

### BACKGROUND

Proper hydration helps maintain body temperature, remove waste, lubricate joints, and is good for overall health. In addition, being well-hydrated also improves sleep quality, cognition, and mood. For professional athletes, hydration is essential for peak athletic performance. Although many people are aware of the importance of proper hydration, it is difficult for individuals to know whether they are consuming the right amount of fluids every day, especially those with a busy schedule or older adults who start to lose their sense of thirst. Also, while some people attempt to manually track their own fluid consumption, this can be tedious and is prone to inaccurate data entries, particularly when those people are busy or prone to forgetting to enter their fluid intake every time.
US 2020/135319 Al discloses an automated medication dispensing system provides for triggering a medication administration message when an inferred event is detected for which a medication administration message is to be sent. The event might be the start, the beginning, or an anticipation of a start, of an eating event, detected by an event detection module from gestures of a user from a set of sensor readings. The message can be a signal to medication dispensing apparatus, and/or a reminder message to the user, an ancillary message to a caregiver, health professional, or others. The medication administration message might comprise a signal to an input of an insulin management system or an input of a meal-aware artificial pancreas. The events might also include a drinking event, a smoking event, a personal hygiene event, and/or a medication related event
US 2011/125063 Al discloses a system for detecting non-verbal acoustic energy generated by a subject. The system includes a sensor mountable on or in a body region of the subject, the sensor being capable of sensing the non-verbal acoustic energy; and a processing unit being capable of processing the non-verbal acoustic energy sensed by the sensor and deriving an activity related signature therefrom, thereby enabling identification of a specific activity associated with the non-verbal acoustic energy.
US 2016/026767 Al discloses an apparatus including a sensor configured to detect a variable characteristic, the variation of the characteristic including variation indicative of an individual swallowing when the sensor is positioned in a neck area of the individual. The apparatus includes a wireless data communication interface configured to receive information related to the characteristic and transmit the information externally. The sensor may be, for example, an acoustic sensor, a piezoelectric sensor, a capacitive sensor, or a pressure sensor. The apparatus may include a sensor interface to sample a signal from the sensor and provide data related to the signal for transmission externally. A system may use the information related to the characteristic to identify eating habits and type of food eaten. Feedback may be provided to the individual to help the individual change their dietary intake and habits.

### SUMMARY

The invention is defined in the independent claims. Optional features are defined in the dependent claims.

### BRIEF DESCRIPTION OF THE DRAWINGS

The accompanying drawings, which are incorporated herein and constitute part of this specification, illustrate exemplary embodiments, and together with the general description given above and the detailed description given below, serve to explain the features of the various embodiments.
FIG. 1 is a schematic diagram illustrating an electronic device in the form of a user equipment working in conjunction with wearables for tracking hydration of a user in accordance with various embodiments.
FIG. 2 is a block diagram illustrating components of an example system in a package for use in an electronic device in accordance with various embodiments.
FIG. 3 is a component block diagram of an example system configured for executed by a processor of electronic device for ensuring a user receives scheduled notifications.
FIGS. 4A, 4B, 4C, 4D, 4E, 4F, 4G, 4H, 4I, and/or 4J show process flow diagrams of example methods for tracking hydration of a user executed by a processor of electronic device according to various embodiments.
FIG. 5 is a process flow diagrams for tracking hydration by monitoring only fluids consumed by a user according to various embodiments.
FIG. 6 is another process flow diagram for tracking hydration by monitoring both fluids and solids consumed by a user according to various embodiments.
FIG. 7 is a component block diagram of a network computing device suitable for use with various embodiments.
FIG. 8 is a component block diagram of a wireless computing device suitable for use with various embodiments.
FIG. 9 is a component block diagram of an example of smart glasses suitable for use with various embodiments.

### DETAILED DESCRIPTION

Various aspects will be described in detail with reference to the accompanying drawings. Wherever possible, the same reference numbers will be used throughout the drawings to refer to the same or like parts. References made to particular examples and embodiments are for illustrative purposes and are not intended to limit the scope of the various aspects or the claims.

Various embodiments provide methods executed by a processor of an electronic device for tracking hydration of a user. Various embodiments may determine an amount of liquid consumed by the user based on a predetermined baseline consumption rate for the user and a number of at least one of sips or swallows by the user that are detected by a sensor coupled to the processor in response to determining that a received indication of consumption by the user involves drinking a liquid. In addition, a hydration level of the user may be determined based on the determined amount of liquid consumed.

As used herein, the term "hydration level" refers to a percentage of body water or fluid in a user's body. An ideal hydration level, may vary based on a number of factors (e.g., age, gender, weight, levels of exercise, exertion, etc.). In general, women require less total hydration than men. For example, the ideal percentage of hydration for adult women may fluctuate between 45 and 60%, while the ideal percentage of hydration for adult men may be between 50 and 65% of total body weight. For athletic body types, ideal hydration levels may need to be 5% higher than the average adult range.

As used herein, the term "electronic device" refers to an electronic device equipped with at least a processor, and memory configured with a contact database. Also, as used herein the term "user equipment" refers to a particular type of electronic device from which a user may receive notifications. Electronic devices, including user equipment, may include any one or all of cellular telephones, smart-phones, portable computing devices, personal or mobile multi-media players, laptop computers, tablet computers, 2-in-1 laptop/table computers, smart-books, ultrabooks, palmtop computers, wireless electronic mail receivers, multimedia Internet-enabled cellular telephones, wearable devices including smart-watches, smart-glasses, ear-pieces (e.g., headphones or ear-buds), smart-contact lenses, augmented/virtual reality devices, entertainment devices (e.g., wireless gaming controllers, music and video players, satellite radios, etc.), exercise equipment, and similar electronic devices that include a memory, and a programmable processor. In various embodiments, electronic devices may be configured with memory and/or storage. Additionally, electronic devices referred to in various example embodiments may be coupled to or include wired or wireless communication capabilities implementing various embodiments, such as network transceiver(s) and antenna(s) configured to communicate with wireless communication networks.

As used herein, the term "smart" in conjunction with a device, refers to a device that includes a processor for automatic operation, for collecting and/or processing of data, and/or may be programmed to perform all or a portion of the operations described with regard to the various embodiments herein. For example, a smart-phone, smart-glasses, smart-contact lenses, smart-watch, smart-ring, smart-necklace, smart-cup, smart-straw, smart-appliances, etc.

The term "system on chip" (SOC) is used herein to refer to a single integrated circuit (IC) chip that contains multiple resources and/or processors integrated on a single substrate. A single SOC may contain circuitry for digital, analog, mixed-signal, and radio-frequency functions. A single SOC may also include any number of general purpose and/or specialized processors (digital signal processors, modem processors, video processors, etc.), memory blocks (e.g., ROM, RAM, Flash, etc.), and resources (e.g., timers, voltage regulators, oscillators, etc.). SOCs may also include software for controlling the integrated resources and processors, as well as for controlling peripheral devices.

The term "system in a package" (SIP) may be used herein to refer to a single module or package that contains multiple resources, computational units, cores and/or processors on two or more IC chips, substrates, or SOCs. For example, a SIP may include a single substrate on which multiple IC chips or semiconductor dies are stacked in a vertical configuration. Similarly, the SIP may include one or more multichip modules (MCMs) on which multiple ICs or semiconductor dies are packaged into a unifying substrate. A SIP may also include multiple independent SOCs coupled together via high speed communication circuitry and packaged in close proximity, such as on a single motherboard or in a single wireless device. The proximity of the SOCs facilitates high speed communications and the sharing of memory and resources.

Various embodiments use one or more electronic devices, such as one or more sensor-equipped wearable electronic devices (also referred to herein as a "wearable") or other sensor-equipped electronic devices, to measure activity associated with individualized drinking rates, as well as eating behaviors, in order to automatically track total liquid consumption and determine user hydration levels. The sensors may be used to detect certain behaviors of a user that are associated with drinking, eating, or other activities and/or conditions. A variety of sensors may be used to detect drinking behaviors by analyzing video images of what a user consumes, as well as detecting other indicators such as sounds, vibrations, muscle activity, and/or pressure changes in a user's ear when taking sips and/or swallowing. For example, to differentiate liquid intake from solid food intake, movement sensors may be used to detect the motion of the lower jaw and/or electromyogram may be used to monitor jaw muscle activity when swallowing, such as to distinguish liquid consumption from solid food that is chewed before swallowing. In addition, or alternatively, wearable cameras may be used to capture images of what a user consumes for analysis. Cameras and/or gas chemical sensors may identify different types of liquids (e.g., coffee, alcohol, juice, water, soda, etc.), solids, and/or combinations thereof. Thermal imaging cameras may measure ambient temperature, the temperature of consumables, the volume of a container holding a liquid versus the volume that is empty (i.e., due to thermal differences between the air and liquid contents), etc.

Certain types of liquids, such as alcohol or coffee, may dehydrate the body due to diuretic effects or make the body retain water due to anti-diuretic effects. Thus, in addition to determining the total amount of liquids consumed, detection of diuretic and/or anti-diuretic consumption may be used to adjust a total hydration level determined for a user.

Sensors may be used to gather information useful for estimating the amount and types of liquids consumed may be incorporated into mobile electronic devices, such as smart-phones and tablets, as well as wearables, such as a pair of smart glasses, ear-pieces (e.g., headphone or ear-buds), other head-worn devices, necklaces, chest straps, watches, bracelets, other wrist-worn device, smart rings, and/or other wearable electronic devices. In addition, the sensors may be incorporated into remote sensing devices, such as smart-appliances or dedicated remote sensors.

In addition to the time of liquid intake and the type of liquid, various embodiments may measure and track more accurately the amount of liquid a user drinks by developing, maintaining, and using a baseline consumption rate of an individual's liquid intake amounts per sip and/or swallow. For example, controlled liquid intake measurements may be taken and recorded for an individual, possibly under different circumstances (e.g., varied temperature, time of day, type of liquid, amount of activity, etc.), through a calibration or learning process, in which the user drinks a predetermined amount of liquid and the sensors calculate how many times the user sips and/or swallows to consume that predetermined amount of liquid. The calibration may further involve the intake of different types of liquids for potentially establishing different baseline consumption rates for each. Such controlled liquid intake measurements use the active participation and input of the user and is thus referred to herein as "active baselining." Active baselining may be particularly useful for initially determining how much the user consumes, on average, with each sip or swallow. The active baselining may also help determine how much the user swallows, on average, relative to each sip or vise-versa. Establishing an active baseline may be useful for situations in which sensors fail to detect or cannot accurately detect what or how much liquid is being consumed by the user.

While a previously determined (i.e., predetermined) baseline consumption rate is useful when sensors cannot accurately detect what or how much liquid is being consumed, sometimes the sensors are able to accurately measure liquid consumption. Thus, rather than using the predetermined baseline consumption rate measure, various embodiments may use sensor measurements only or mainly to determine how much liquid was consumed. In addition, when the sensors are able to accurately measure liquid consumption, a processor may use that measure of liquid consumption to determine, verify, and/or update the baseline consumption rate without the user manually or actively entering information about what was consumed, which is herein referred to as "passive baselining." Passive baselining may be useful for situations in which sensors are reliably able to measure and determine what or how much liquid is being consumed by the user. For example, if a user has a habit of having the same drink every day or drinks from a can or bottle of a defined amount, clear glass, or smart-cup that provides a reliable measurement of the amount of liquid consumed. In this way, once an active baseline is established for a user, passive baselining may provide a continuous calibration or refinement of the baseline for more accurately estimating the total hydration of a user.

Various embodiments may analyze what is consumed by a user to determine whether the user is consuming solid foods, liquids, something in between (e.g., yogurt), or a combination thereof. Besides liquid intake, the water content of food often contributes 20%-30% of an individual's total water intake. As the water content of foods is highly variable and people have different eating habits, the amount of water obtained from food may vary between individuals, which will impact their total hydration levels. Thus, various embodiments may monitor (e.g., with a camera on a wearable) and account for the amount and types of food (e.g. vegetables, fruit, fish, meat, grains, etc.) that a user eats. Using a look up table or database, an average water content may be determined for the amount and type of food detected. In cases in which the type of food cannot be automatically detected, various embodiments may receive manual input from the user identifying the food being eaten. By analyzing a user's diet pattern, an electronic device may provide the user recommendations and feedback to help the user achieve proper and/or better hydration.

Various embodiments may additionally apply a contextual analysis to more accurately determine a current hydration level of a user. The contextual analysis may be used to refine baseline measurements, and thus improve the accuracy of how much liquid the user has consumed and is likely to retain in a particular context. For example, various embodiments may account for perspiration from exercise and external temperature and humidity when estimating the hydration level of a user. In this way, a processor may consider a totality of information received from sensors to account not only for how much liquid was consumed, but also how much liquid is lost or not retained for the sake of determining total hydration.

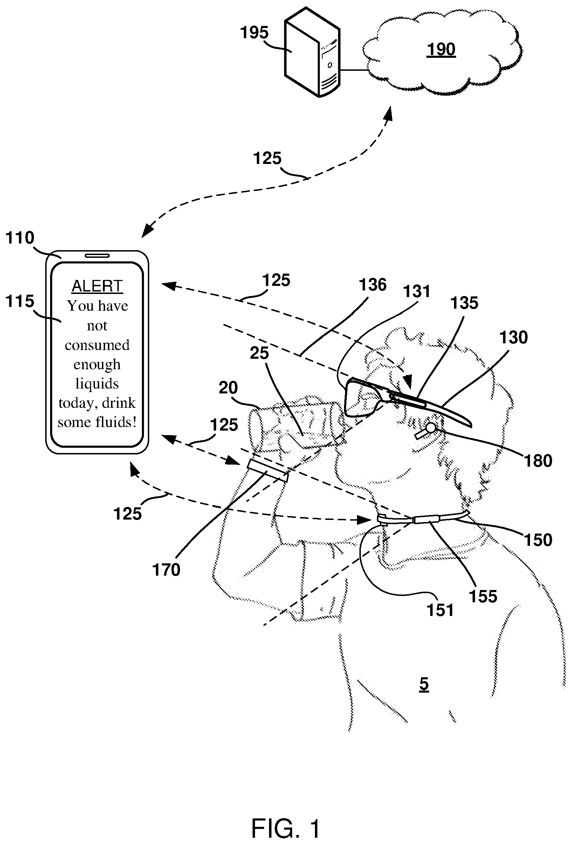

FIG. 1 illustrates actions of various embodiments of a user 5 consuming a liquid 25 while various sensors take measurements that are processed by an electronic device in the form of a user equipment 110 to estimate a hydration level of the user. The illustrated user equipment 110 is in the form of a cellular telephone (i.e., a smart-phone), although other forms of electronic devices may be used (e.g., tablets, personal computers, exercise machines, etc.). Various devices having sensors including wearables, such as smart-glasses 130, a sensor-equipped neck-strap 150, a smart-watch 170, and ear-piece 180. The user equipment 110 may be configured to communicate through wireless connections 125 (e.g., Wi-Fi, Bluetooth, cellular, etc.) with remote computing devices (e.g., a server 195) through a wireless network 190, which may be supported by a wireless local area network router (not shown), such as a Wi-Fi wireless router, or a cellular network base station.

In various embodiments, the user equipment 110 may determine an amount of liquid 25 consumed by the user 5 in response to determining that an indication of consumption by the user 5 received from a sensor involves drinking a liquid. In some embodiments, the user equipment (or other electronic device) may determine the amount of liquid consumed based on a predetermined baseline consumption rate for the user 5 and a number of sips and/or swallows by the user 5 as detected based on data provide by one or more sensors, such as smart-glasses 130, a sensor-equipped neck-strap 150, and a smart-watch 170. The indication of liquid consumption sips and/or swallows may be determined by the user equipment 110 by correlating information from the one or more sensors (e.g., wearables) with patterns of swallowing determined during calibration.

Smart-glasses 130 are a form of wearable that may include a built-in camera 135 as well as heads-up display or augmented reality features on or near the front lenses 131. Like other sensor devices providing information to the user equipment 110, the smart-glasses 130 may collect information from external sensors, such as a head-mounted camera 135 with a field of view 136, an electromyogram (e.g., in the arms of the frame), microphone, thermometer, and/or internal sensors, such an inertial measurement unit (IMU), proximity/motion sensor, light sensor, lidar, gas sensor, etc. The electromyogram may detect muscle movements associated with sipping or swallowing. Microphones may detect sipping and/or swallowing sounds, as well as the sounds made by some carbonated drink, particularly when opened. The thermometer may register the user's temperature and/or an ambient temperature around the user 5, which may provide contextual information relevant to determining the user's total hydration levels. When a user is very hot, that user may take bigger sips (e.g., gulps) of liquid. Similarly, when the air temperature is hot, users may drink more rapidly. Thus, based on previous calibrations for the user, the estimated amount of liquid consumed with each sip may be adjusted based on the user's temperature and/or the ambient temperature. In addition, sensors like a laser thermometer or a thermal imaging camera (not shown) may be used to detect the temperature of foods and/or liquids consumed by the user 5. Users consuming very hot or very cold foods or liquids tend to consume smaller amounts. Thus, the estimated amount of liquid consumed with each sip may be adjusted based on the temperature of the substance being consumed. In this way, the temperature of the user, ambient temperature, and/or the temperature of the food or liquid being consumed may provide contextual information relevant to determining the user's consumption and thus the user's total hydration levels. An IMU may detect drinks 25 or food brought to the mouth, and detect the angles of tilt of a glass 20 and the wearer's head, which may be used to determine remaining contents of a container and/or the size of sips, and thus how much was consumed. A gas sensor may detect the type of food or liquid being consumed. The smart-glasses 130 may support wireless technologies like Bluetooth of Wi-Fi, enabling communications through a wireless communication link 125, such as to the user equipment 110 and/or other wearables (e.g., 150, 170). In addition, the smart-glasses 130 may control or retrieve data from the other wearables (e.g., 150, 170) and/or remote computing devices (110, 195).

Individual sensors, such as a camera 135, may image drinks 25 (i.e., liquids) or food (i.e., solids and/or mixed solids with liquids) brought to the mouth (e.g., in a glass 20), to enable determining through image processing the type of drink or food that is being consumed, how much is being consumed, and perhaps how much liquid is not consumed (e.g., by measuring the remaining contents of a container).

In some embodiments, the smart-glasses 130 may operate independently, with a mobile operating system, and may provide feedback to the user 5 through an augmented reality or heads-up display on the front lenses 131, through an audible or haptic feedback, if available, and/or through the wireless communication link 125 to other electronic devices.

A sensor-equipped neck-strap 150 may be in the form of a necklace, choker, or other neck-worn apparel/accessory. A sensor-equipped neck-strap 150 may collect information from external sensors, such as a pressure sensor 151 and/or a neck-mounted camera 155, microphone, and/or internal sensors, such an IMU, proximity/motion sensor, gas sensor, etc. The sensor-equipped neck-strap 150 may operate independently, with a mobile operating system, to provide feedback to the user 5 through vibrations, sound, and/or electric stimulus along a band of the sensor-equipped neck-strap 150. Also, the sensor-equipped neck-strap 150 may support wireless technologies like Bluetooth and Wi-Fi, enabling communications through a wireless communication link 125, such as the user equipment 110 and/or other wearables (e.g., 130, 170, 180). Thus, the sensor-equipped neck-strap 150 may provide feedback to the user 5 through the wireless communication link 125. In addition, the sensor-equipped neck-strap 150 may control or retrieve data from the other wearables (e.g., 130, 170, 180) and/or remote computing devices (110, 195).

The smart-watch 170 may collect information from external sensors, such as one or more sensors facing and/or in contact with the user's wrist (i.e., skin), and/or internal sensors, such as an IMU, proximity/motion sensor, light sensor, gas sensor, camera, etc. The smart-watch 170 may operate with a mobile operating system to provide feedback to the user 5 through a watch-face display, vibrations, and/or sound. The smart-watch 170 may support wireless technologies like Bluetooth and Wi-Fi, enabling communications through a wireless communication link 125, such as the user equipment 110 and/or other wearables (e.g., 130, 150, 180). Thus, the smart-watch 170 may provide feedback to the user 5 through the wireless communication link 125. In addition, the smart-watch 170 may control or retrieve data from the other wearables (e.g., 130, 150, 180) and/or remote computing devices (110, 195).

The ear-piece 180 may collect information from external sensors, such as one or more sensors facing and/or in contact with the user's ear or cheek (i.e., skin), and/or internal sensors, such as an IMU, proximity/motion sensor, light sensor, gas sensor, camera, microphone, etc. The ear-piece 180 may operate with a mobile operating system to provide feedback to the user 5 through vibrations and/or sound. The ear-piece 180 may support wireless technologies like Bluetooth and Wi-Fi, enabling communications through a wireless communication link 125, such as the user equipment 110 and/or other wearables (e.g., 130, 150, 170). Thus, the ear-piece 180 may provide feedback to the user 5 through the wireless communication link 125. In addition, the ear-piece 180 may control or retrieve data from the other wearables (e.g., 130, 150, 170) and/or remote computing devices (110, 195).

The user equipment 110 and/or the wearables 130, 150, 170, 180 may include other or additional sensors, such as the camera, microphone, IMU, clocks, electromyograms, gas sensors, pressure sensor, proximity/motion sensor, light sensor, and thermometer. Although three wearables 130, 150, 170, 180 are illustrated in FIG. 1, various embodiments may include fewer or a greater number of wearables and/or remote computing devices, including one or more different types of wearables and/or electronic device not shown in FIG. 1. For example, various embodiments may include remote computing devices that act like accessories, such as a smart-cup or smart-straw that may be used to detect how much fluid the user 5 consumes.

The user equipment 110 may use conventional functions applied to the determination of how much fluid was consumed, such as a clock/timer that may provide a measure of how long a container is held to the mouth, which may indicate how much liquid poured out, if combined with a tilt angle measurement.

In addition to determining a current hydration level of a user by estimating the amount of water consumed, the user equipment 110 may be configured to provide feedback to the user 5 through the display 115, such as an alert that may read, "ALERT, You have not consumed enough liquids today, drink some fluids!"

In some embodiments, the predetermined baseline consumption rate may be determined for the user based on a previously counted number of at least one of sips or swallows detected by the sensor when the user consumed a known amount of liquid. Additionally, or alternatively, the predetermined baseline consumption rate may be determined for the user based on a manual user input indicating at least one of the known amount of liquid or the number of sips or swallows it took to consume the known amount of liquid. As a further addition or further alternative, the predetermined baseline consumption rate may be determined for the user based on a context in which the liquid was consumed. For example, the user may have a habit of drinking more slowly in the morning, which may be factored into the predetermined baseline consumption rate for mornings. Similarly, the user may tend to drink bigger sips when at the office, when exercising, or when the ambient temperature is hot and/or the humidity is high, which may be factored into the predetermined baseline consumption rate under those circumstances.

In some embodiments, an update to a previously determined baseline consumption rate may be determined for the user based on liquid consumption information from at least one of the sensor, a user input, or a context in which the liquid was consumed. The predetermined baseline consumption rate may be based on the update to the previously determined baseline consumption rate for the user.

In some embodiments, a processor of the user equipment 110 may receive contextual information indicating a context in which the number of at least one of sips or swallows were performed by the user. Also, the hydration level of the user may further be determined based in part on the context in which the number of at least one of sips or swallows were performed by the user.

Some embodiments may determine whether the user is consuming solids in combination with the amount of liquid consumed. Also, determining the amount of liquid consumed by the user may be based on the number of sips by the user in response to determining that the user is consuming solids in combination with liquid.

The electronic device (e.g., user equipment 110) receives an indication of the type of liquid consumed by the user. Using such information, the electronic device determines a hydration level of the user further based on the type of liquid consumed by the user. For example, if the received indication of the type of liquid consumed by the user indicates that the liquid consumed by the user includes at least one of a diuretic or dehydrating substance, the electronic device may determine a hydration level of the user based on the determined amount of liquid consumed with a decrease adjustment to the hydration level to account for the diuretic or dehydrating substance indicated in the received indication. As another example, if the received indication of the type of liquid consumed by the user indicates that the liquid consumed by the user includes an anti-diuretic, the electronic device may determine a hydration level of the user based on the determined amount of liquid consumed with an increase adjustment to account for the anti-diuretic is indicated. Similarly, if the received indication of the type of liquid consumed by the user indicates that the liquid is a medication having either diuretic or anti-diuretic properties, the determined hydration level of the user may be adjusted accordingly.

In some embodiments, the electronic device (e.g., user equipment 110) may receive food consumption information associated with food consumed by the user; and determine the hydration level of the user further based in part on the received food consumption information. In addition, the hydration level of the user may be further determined based in part on the received food consumption information.

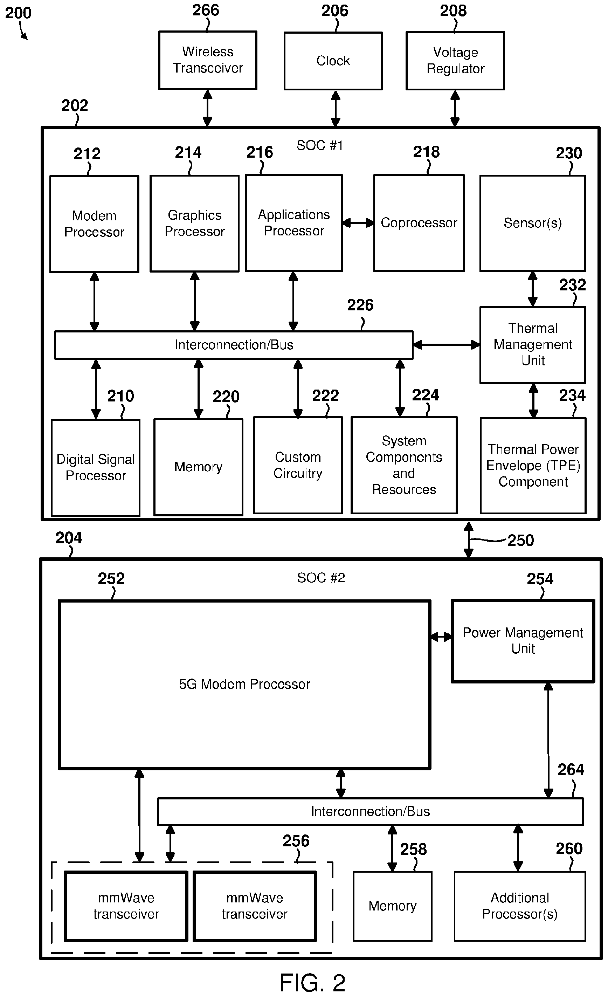

Various embodiments may be implemented in various types of user equipment and electronic devices using a number of single processor and multiprocessor computer systems, including a system-on-chip (SOC) or system in a package (SIP). FIG. 2 illustrates an example computing system or SIP 200 architecture that may be used in an electronic device, such as the electronic device (e.g., user equipment 110) and/or one or more of the wearables (e.g., 130, 150, 170, etc.) implementing the various embodiments.

With reference to FIGS. 1 and 2, the illustrated example SIP 200 includes a two SOCs 202, 204, a clock 206, a voltage regulator 208, and a wireless transceiver 266. In some embodiments, the first SOC 202 operates as central processing unit (CPU) of the wireless device that carries out the instructions of software application programs by performing the arithmetic, logical, control and input/output (I/O) operations specified by the instructions. In some embodiments, the second SOC 204 may operate as a specialized processing unit. For example, the second SOC 204 may operate as a specialized 5G processing unit responsible for managing high volume, high speed (e.g., 5 Gbps, etc.), and/or very high frequency short wave length (e.g., 28 GHz mmWave spectrum, etc.) communications.

The first SOC 202 may include a digital signal processor (DSP) 210, a modem processor 212, a graphics processor 214, an application processor 216, one or more coprocessors 218 (e.g., vector co-processor) connected to one or more of the processors, memory 220, custom circuitry 222, system components and resources 224, an interconnection/bus module 226, one or more sensors 230 (e.g., thermal sensors, motion sensors, proximity sensors, a multimeter, etc.), a thermal management unit 232, and a thermal power envelope (TPE) component 234. The second SOC 204 may include a 5G modem processor 252, a power management unit 254, an interconnection/bus module 264, a plurality of mmWave transceivers 256, memory 258, and various additional processors 260, such as an applications processor, packet processor, etc.

Each processor 210, 212, 214, 216, 218, 252, 260 may include one or more cores, and each processor/core may perform operations independent of the other processors/cores. For example, the first SOC 202 may include a processor that executes a first type of operating system (e.g., FreeBSD, LINUX, OS X, etc.) and a processor that executes a second type of operating system (e.g., MICROSOFT WINDOWS 10). In addition, any or all of the processors 210, 212, 214, 216, 218, 252, 260 may be included as part of a processor cluster architecture (e.g., a synchronous processor cluster architecture, an asynchronous or heterogeneous processor cluster architecture, etc.).

The first and second SOC 202, 204 may include various system components, resources and custom circuitry for managing sensor data, analog-to-digital conversions, wireless data transmissions, and for performing other specialized operations, such as decoding data packets and processing encoded audio and video signals for rendering in a web browser. For example, the system components and resources 224 of the first SOC 202 may include power amplifiers, voltage regulators, oscillators, phase-locked loops, peripheral bridges, data controllers, memory controllers, system controllers, access ports, timers, and other similar components used to support the processors and software clients running on a wireless device. The system components and resources 224 and/or custom circuitry 222 may also include circuitry to interface with peripheral devices, such as cameras, electronic displays, wireless communication devices, external memory chips, etc.

The first and second SOC 202, 204 may communicate via interconnection/bus module 250. The various processors 210, 212, 214, 216, 218, may be interconnected to one or more memory elements 220, system components and resources 224, and custom circuitry 222, and a thermal management unit 232 via an interconnection/bus module 226. Similarly, the processor 252 may be interconnected to the power management unit 254, the mmWave transceivers 256, memory 258, and various additional processors 260 via the interconnection/bus module 264. The interconnection/bus module 226, 250, 264 may include an array of reconfigurable logic gates and/or implement a bus architecture (e.g., CoreConnect, AMBA, etc.). Communications may be provided by advanced interconnects, such as high-performance networks-on chip (NoCs).

The first and/or second SOCs 202, 204 may further include an input/output module (not illustrated) for communicating with resources external to the SOC, such as a clock 206 and a voltage regulator 208. Resources external to the SOC (e.g., clock 206, voltage regulator 208) may be shared by two or more of the internal SOC processors/cores.

In addition to the example SIP 200 discussed above, various embodiments may be implemented in a wide variety of computing systems, which may include a single processor, multiple processors, multicore processors, or any combination thereof.

In some embodiments, only one SOC (e.g., 202) may be used in a less capable electronic device, such as wearables (e.g., 130, 150, 170, etc.) that are configured to provide sensor information to a more capable electronic device, such as a smart phone 110. In such embodiments, communication capabilities of the wearable (e.g., 130, 150, 170, etc.) may be limited to a short-range communication link, such as Bluetooth or Wi-Fi, in which case the 5G capable SOC 204 may not be included in the processing system of the wearable.

As used herein, the terms "component," "system," "unit," "module," and the like include a computer-related entity, such as, but not limited to, hardware, firmware, a combination of hardware and software, software, or software in execution, which are configured to perform particular operations or functions. For example, a component may be, but is not limited to, a process running on a processor, a processor, an object, an executable, a thread of execution, a program, and/or a computer. By way of illustration, both an application running on a communication device and the communication device may be referred to as a component. One or more components may reside within a process and/or thread of execution and a component may be localized on one processor or core and/or distributed between two or more processors or cores. In addition, these components may execute from various non-transitory computer readable media having various instructions and/or data structures stored thereon. Components may communicate by way of local and/or remote processes, function or procedure calls, electronic signals, data packets, memory read/writes, and other known computer, processor, and/or process related communication methodologies.

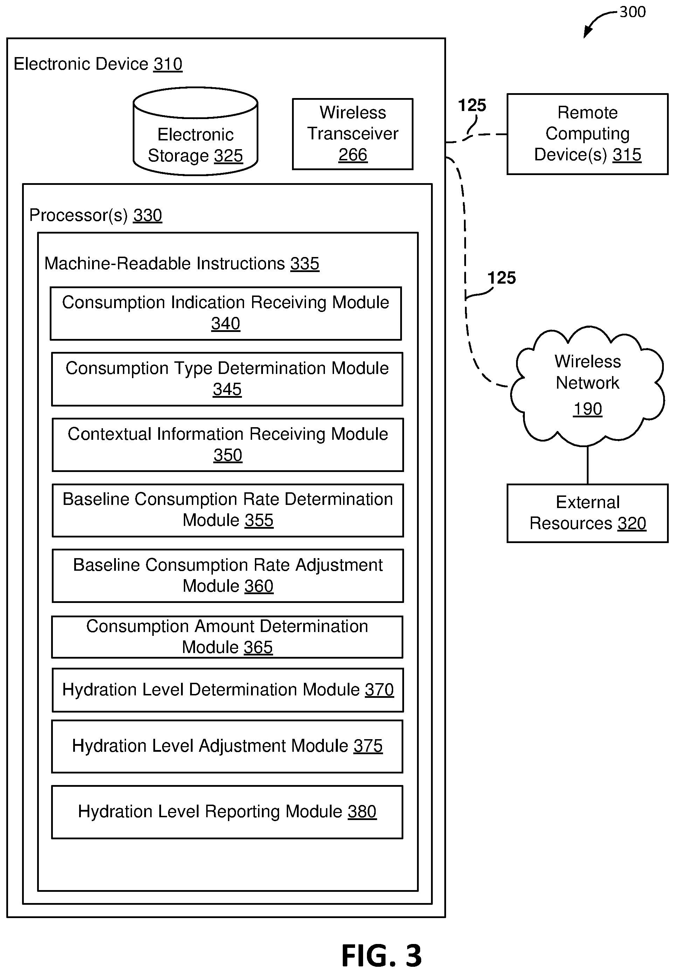

FIG. 3 is a component block diagram illustrating a system 300 configured for tracking hydration of a user executed by a processor of an electronic device 310 in accordance with various embodiments. With reference to FIGS. 1-3, the system 300 may include the electronic device 310 (e.g., user equipment 110) and be configured to communicate with one or more remote computing device(s) 315 (e.g., 130, 150, 170, in FIG. 1) or other electronic devices via a local area wireless network router (e.g., Wi-Fi, Bluetooth, Ant, etc.), which may be coupled to the Internet. The electronic device 310 (e.g., user equipment 110) may also be configured to communicate with external resources 320 (e.g., server 195) via a wireless network 190, such as a cellular wireless communication network.

The electronic device 310 (e.g., user equipment 110) may include electronic storage 325, one or more processors 330, a wireless transceiver 266, and other components. The electronic device 310 may include communication lines, or ports to enable the exchange of information with a network and/or other computing platforms. Illustration of the electronic device 310 in FIG. 3 is not intended to be limiting. The electronic device 310 may include a plurality of hardware, software, and/or firmware components operating together to provide the functionality attributed herein to the electronic device 310.

Electronic storage 325 may include non-transitory storage media that electronically stores information. The electronic storage media of electronic storage 325 may include one or both of system storage that is provided integrally (i.e., substantially non-removable) with the electronic device 310 (e.g., user equipment 110) and/or removable storage that is removably connectable to the electronic device 310 via, for example, a port (e.g., a universal serial bus (USB) port, a firewire port, etc.) or a drive (e.g., a disk drive, etc.). Electronic storage 325 may include one or more of optically readable storage media (e.g., optical disks, etc.), magnetically readable storage media (e.g., magnetic tape, magnetic hard drive, floppy drive, etc.), electrical charge-based storage media (e.g., EEPROM, RAM, etc.), solid-state storage media (e.g., flash drive, etc.), and/or other electronically readable storage media. Electronic storage 325 may include one or more virtual storage resources (e.g., cloud storage, a virtual private network, and/or other virtual storage resources). Electronic storage 325 may store software algorithms, information determined by processor(s) 330, information received from the user equipment 310, information received from remote platform(s) 304, and/or other information that enables the electronic device 310 to function as described herein.

Processor(s) 330 may be configured to provide information processing capabilities in the electronic device 310 (e.g., user equipment 110). As such, processor(s) 330 may include one or more of a digital processor, an analog processor, a digital circuit designed to process information, an analog circuit designed to process information, a state machine, and/or other mechanisms for electronically processing information. Although processor(s) 330 is shown in FIG. 3 as a single entity, this is for illustrative purposes only. In some embodiments, processor(s) 330 may include a plurality of processing units. These processing units may be physically located within the same device, or processor(s) 330 may represent processing functionality of a plurality of devices operating in coordination.

The electronic device 310 (e.g., user equipment 110) may be configured by machine-readable instructions 335, which may include one or more instruction modules. The instruction modules may include computer program modules. In particular, the instruction modules may include one or more of a consumption indication receiving module 340, a consumption type determination module 345, a contextual information receiving module 350, an baseline consumption rate determination module 355, a baseline consumption rate adjustment module 360, a consumption amount determination module 365, a hydration level determination module 370, a hydration level adjustment module 375, a hydration level reporting module 380, and/or other instruction modules.

The consumption indication receiving module 340 may be configured to receive a consumption indication from one or more sensors of the electronic device 310 (e.g., user equipment 110) and/or local computing devices within the vicinity of the electronic device 310 (e.g., the smart-glasses 130, sensor-equipped neck-strap 150, and smart-watch 170, etc.). The sensors may detect the user performing certain types of actions associated with consumption of drinks/food and/or may detect the presence of a consumable (i.e., a drink and/or food). By way of a non-limiting example, the consumption indication may come from cameras, lidar, light sensors, microphones, IMUs, electromyograms, pressure sensors, proximity/motion sensors, and gas sensors. Cameras and lidar may detect drinks or food brought to the mouth. Microphones may detect sipping and/or swallowing sounds, as well as the sound of a can or carbonated drink being opened. IMUs may detect drinks or food brought to the mouth. Electromyograms may detect muscle movements associated with sipping and/or swallowing. Gas sensors may detect when a food and/or liquid is near the user's mouth (i.e., being or ready to be consumed). Smart-cups or smart-straws may detect when liquids are being dispensed.

A processor (e.g., 210, 212, 214, 216, 218, 252, 260) of the electronic device may receive consumption indication information directly from onboard sensors and/or use one or more transceivers (e.g., 256, 266) for detecting available wireless connections 125 (e.g., Wi-Fi, Bluetooth, cellular, etc.). Also, the consumption indication receiving module 340 may be configured to determine whether a detected communication link is available to a wearable or other remote computing device.

The consumption type determination module 345 may be configured to determine whether the consumption indication (i.e., received by the consumption indication receiving module 340) suggests the user is eating and/or drinking. In addition, the consumption type determination module 345 may be configured to determine the type of food or the type of drink that is being consumed. By way of a non-limiting example, the camera(s) and/or lidar(s) may collect imaging that identifies the consumable, the microphones may collect sounds (e.g., a carbonated beverage can opening), electromyograms may collect indications of muscle movements (e.g., associated with chewing versus sipping), and smart-devices may provide specific information about what generally or specifically is being consumed. The processor (e.g., 210, 212, 214, 216, 218, 252, 260) may use details about what is being consumed or how it is being consumed to determine whether the user is eating and/or drinking. The determination of what type of consumable is being consumed (i.e., drink versus food, food mix, or specifically the type of food, drink, or food/drink mix that is being consumed) may be directly from an identification of the food, drink, or food/drink mix itself or indirectly from the container, plate, or other object used to deliver the food, drink, or combination thereof. In addition, depending upon the level of detail included in the consumption indication (i.e., received by the consumption indication receiving module 340), the consumption type determination module 345 may be configured to determine more precisely what is being or has been consumed. For example, the consumption indication may identify a consumed food and/or beverage as containing a diuretic or anti-diuretic, which may affect the user's total hydration.

In order to determine the type of consumable being consumed, the consumption type determination module 345 may also access a database, containing one or more data records, stored in memory (e.g., 220, 258, 325) or from a remote source, such as a remote system (e.g., 315) or external resources (e.g., 320) using a transceiver (e.g., 256, 266) and related components. The processor of the electronic device may access the data records to compare previously stored information regarding sensor data with data received from sensors in order to determine what the received consumption indications represent. For example, a lookup table may provide information used to determine the type of consumable being detected and/or even specific details thereof. Alternatively, a gas sensor may be configured to detect gas, which the processor may use to determine the consumable or type of consumable that is being consumed. Thus, the consumption type determination module 345 may determine whether the user is consuming liquids, solids, or solids in combination with some liquid. The consumption type determine module 345 determines a type of liquid being consumed. The consumption type determine module may determine whether that liquid has diuretic or anti-diuretic properties, which may be determined from information stored in memory. Alternatively or additionally, the consumption type determination module 345 may determine the food or food/liquid mixture that is being consumed, along with how much hydration the consumable provides the user, and possibly whether that consumable has diuretic or anti-diuretic properties.

The contextual information receiving module 350 may be configured to receive contextual information from one or more sources for considering a totality of information available about what is being consumed. Contextual information about what is being consumed and how it is being consumed may be used to adjust determinations about how much liquid was consumed and total hydration levels. Contextual information my include one or more temperatures (e.g., the user's body temperature, the temperature of the consumable, and/or the ambient temperature and/or humidity). In addition, the contextual information may include information about the environment, the time of day, or information about the user's activity levels or wellness that may influence a total hydration determination. In some embodiments, the received contextual information may indicate a context in which the number of at least one of sips or swallows were performed by the user.

In some embodiments, the context in which the number of at least one of sips or swallows were performed by the user may include recent past events or conditions that may impact the user's baseline consumption rate. For example, the user may have just finished exercising or may have just come inside to shelter from extreme heat outside. That user may no longer be exercising or exposed to the extreme heat when drink and/or eating, but the user's body may still feel the influence of such recent past events or conditions, the effects of which may linger for some time. In this way, the context in which a user consumes liquids, solids, or a solid/liquid mix may reflect past events or conditions still influencing the user's baseline consumption rate at the time of consumption.

The temperature and/or composition of the liquid may affect how quickly or slowly a user consumed it. For example, hot coffee is generally sipped more slowly than cool ice tea. While the exact temperature of a liquid or other consumable is helpful, a range of temperatures may be useful for estimating a baseline consumption rate. For example, liquids in the high, medium, or low temperature ranges may be associated with a baseline consumption rate suitable to that respective temperature range. Also, the nature of the liquid, such as whether it is carbonated, viscous, or has solids in it (e.g., ice), may affect the user's consumption rate. Contents of a liquid, like ice, may be detected visually, but may also be determined based on sounds detected by microphones (e.g., ice clinking in a glass or against a user's teeth). Further, a user's manual input may provide information about the contents and/or temperature of consumables.

By way of a non-limiting example, contextual information may be received from sensors that provide information to the electronic device (e.g., user equipment 110) and/or local computing devices within the vicinity of the electronic device 310 (e.g., the smart-glasses 130, sensor-equipped neck-strap 150, and smart-watch 170, etc.). The contextual information receiving module 350 may also receive contextual information by accessing a database, containing one or more data records, stored in local memory (e.g., 220, 258, 325) or from a remote source, such as a remote system (e.g., 315) or external resources (e.g., 320) using a transceiver (e.g., 256, 266) and related components.

The baseline consumption rate determination module 355 may be configured to determine a baseline consumption rate for the user. In some embodiments, the baseline consumption rate may be determined based on a previously counted number of at least one of sips or swallows detected by a sensor (i.e., sensor-detected parameters) while the user consumes a known amount of liquid (e.g., water). In some other embodiments, the baseline consumption rate may be determined based on a manual user input indicating at least one of the known amount of liquid or the number of sips or swallows it took the user to consume the known amount of liquid. In some embodiments, the baseline consumption rate may be determined based on a context (i.e., contextual information) of the liquid consumed (e.g., obtained by the contextual information receiving module 350). In some embodiments, the baseline consumption rate may be determined based on a combination of the sensor-detected parameters, a manual user input, or the contextual information.

During an initial calibration, the user may be instructed to consume a specified volume of liquid and provide an indication when the liquid is completely consumed. An electronic device may process information received from various sensors and calibrate or correlate that information to the number of sips and/or swallows that it took for the user to consume the liquid during that initial calibration. The number of sips and/or swallows associated with consuming that liquid, as well as the sensor data that was gathered while the user was consuming the liquid, may be used as a baseline consumption rate as well as a baseline sensor calibration. For example, a processor of the electronic device may analyze the sensor data received while the user is consuming the liquid and correlate various sensor readings to sips or swallows knowing the number of sips or swallows that were made by the user during that time. Thus, the calibration process not only calibrates the average amount of liquid consumed per slip or swallow but may correlate various sensor data to the detection of sips or swallows. Again, such calibration of volume-to-sips/swallow and correlation of sensor data to sips or swallows may depend upon the type of liquid being consumed, which may require performing the calibration process for various types of liquids that the user typically consumes. The user may be prompted to indicate or confirm what liquid is being consumed as part of active baselining. The same procedure may be applied to determine a baseline consumption rate as well as sensor calibration for different liquids, liquids at different temperatures, and/or liquids of different consistencies (e.g., viscous, bubbly, creamy, mixed with solids, etc.). In addition, the same calibration process may be used for the same consumable in different contexts (e.g., different ambient temperature/humidity, while exercising, different times of day, different locations, etc.). Once a calibration process has been completed, the results may be stored in memory in the form of lookup tables, and the processor may use the lookup tables for determining amounts of liquid consumed during normal operations.

Alternatively or additionally, passive baselining may be used for the initial calibration under certain circumstances. For example, using a standard size drink container, such as a 12 oz. can, the processor may detect events, such as when the can is opened (e.g., using the microphone), and then determine the number of sips or swallows taken when a finishing event is detected, such as the sounds associated with a last sip or the hollow sound the can makes when placed on a surface, the crimpling sound made when the can is crushed, and/or a sound associated with disposing of the can in the trash.

Determining a baseline consumption rate, using the baseline consumption rate determination module 355, may allow a user to forego wearing most wearables and/or sensors every time they eat or drink. Once the baseline consumption rate for a user is determined, the user may consume liquids and/or solids and the determined baseline consumption rate may be used to determine how much was consumed, rather than needing many wearables and/or sensors for that determination. For example, when calculating the baseline consumption rate, the processor may use inputs from various sensors to determine a number of sips the user is taking and how much liquid is consumed with each sip. Thereafter, once a baseline is determined, the processor may only need input from fewer sensors that determine the number of sips taken, but may use the amount of liquid consumed per sip from the previously determined baseline consumption rate.

By way of a non-limiting example, the processor (e.g., 210, 212, 214, 216, 218, 252, 260) of the electronic device may determine the initial baseline consumption rate using active and/or passive baseline determination techniques. Manual entry of consumption or context information by the user may be helpful for baselining, particularly during the initial calibration process, to ensure the measured number of sips and/or swallows is compared accurately against the proper volume, type, temperature, and consistency of liquid. However, automatic entry of consumption or context information by sensors may also or alternatively be used for the initial calibration process to determine the baseline consumption rate of a user. In addition, the initial baseline consumption rate may take into account contextual factors that may have influenced the initial baseline consumption rate, such as temperature, the environment, the time of day, or information about the user's activity levels, age, sex, weight, or health conditions (e.g., diabetic) that may influence a total hydration determination.

In order to determine a baseline consumption rate, the baseline consumption rate determination module 355 may receive information from sensors and/or access a database, containing one or more data records. The records may be stored in a local memory (e.g., 220, 258, 325) or received from a remote source, such as a remote system (e.g., 315) or external resources (e.g., 320) using a transceiver (e.g., 256, 266) and related components. The sensors may similarly be part of the electronic device or received from a remote source, such as a remote system (e.g., 315) or external resources (e.g., 320) using a transceiver (e.g., 256, 266) and related components.

The baseline consumption rate adjustment module 360 may be configured to determine updates (i.e., a recalibration) to and change a previously determined baseline consumption rate. The determined updates may be based on new consumption information from one or more sensors under circumstances in which the previously determined baseline consumption rate is not necessary to determine the update. A baseline consumption rate for a specific drink may be established and recorded, but subsequently the processor may detect a change in that baseline consumption rate. The baseline consumption rate adjustment module 360 may be used to calculate and record the change. For example, the processor may prompt a user to indicate or confirm when the user is consuming something for which the baseline consumption rate may need an update. If a measured baseline consumption rate is far enough from the recorded baseline consumption rate, the processor may immediately initiate a recalibration routine using the baseline consumption rate adjustment module 360. The processor may alternatively maintain a rolling average of the baseline consumption rate for a drink and prompt the user when that rolling average changes beyond a threshold. The prompt may ask the user for confirmation of a calculated guess (i.e., an estimate) regarding a number of sips and/or swallows taken to consume a known amount of liquid in order to obtain feedback and provide further calibration for generating the update to the previously determined baseline consumption rate for the user. The calculated guess may be based on a different liquid when no baseline consumption rate is available for the liquid being consumed and thus may benefit from user feedback that may lead to a more accurate baseline consumption rate for the new liquid being consumed. Various embodiments may apply machine learning to determine updates, which may occur from time to time, and identify user habits of consumption at certain times of day or certain days of the week that may be useful for determining what and how much is consumed (e.g., a 16 ounce cup of coffee every morning). The processor may correlate contextual information to the determined update. For example, circumstance in which the user has a temperature, observes dark or yellow urine, has been unusually active, or when consumption takes place on a hot or very cold day may be correlated to the determined update for when similar circumstances occur in the future.

In addition, the processor may receive input from a user indicating that an update is needed. For example, after issuing an alert that the user is dehydrated and needs to drink some fluids, the processor may receive a user input indicating the user is not dehydrated, in which case the processor may initiate a recalibration routine for updating one or more previously determined baseline consumption rates. The user may have additional information that confirms he/she is not dehydrated (e.g., frequent urination and/or very light or almost clear urine color). Alternatively, the user may manually initiate the recalibration routine.

The baseline consumption rate adjustment module 360 may enable a user to wear fewer wearables and/or sensors every time they eat or drink. Occasionally when the user does wear a greater number of wearables and/or sensors, the baseline consumption rate adjustment module 360 may be used to recalibrate and update one or more baseline consumption rates stored in memory. For example, the processor may prompt the user from time to time to run a new training routine that involves the user using a smart-cup or smart-straw (i.e., an additional sensor). Similarly, the processor may prompt the user to wear one or more additional wearables and/or sensors during the new training routine. The new training routine may include the consumption of just one cup or may include the consumption of more than one cup at different times and/or on different days.

By way of a non-limiting example, the processor (e.g., 210, 212, 214, 216, 218, 252, 260) of the electronic device (e.g., user equipment 110) may determine an update to a previous baseline consumption rate using active and/or passive baseline determination techniques. In order to determine one or more updates to previous baseline consumption rates, the baseline consumption rate adjustment module 360 may receive information from sensors and/or access a database, containing one or more data records. The records may be stored in a local memory (e.g., 220, 258, 325) or received from a remote source, such as a remote system (e.g., 315) or external resources (e.g., 320) using a transceiver (e.g., 256, 266) and related components. The sensors may similarly be part of the electronic device or received from a remote source, such as a remote system (e.g., 315) or external resources (e.g., 320) using a transceiver (e.g., 256, 266) and related components.

The consumption amount determination module 365 may be configured to determine an amount of liquid consumed by the user based on the predetermined baseline consumption rate (determined by the baseline consumption rate determination module 355 and/or updated by the baseline consumption rate adjustment module 360) and a number of at least one of sips or swallows by the user that are detected by one or more sensors coupled to the processor. In various embodiments, a clock may provide a measure of how long a container is held to the mouth, which may indicate how much liquid poured out, if combined with a tilt angle measurement measured by other sensors, such as an IMU. In some embodiments, the consumption amount determination module 365 may measure how much is consumed through image analysis of camera, thermal, and/or lidar imaging. Alternatively, the consumption amount determination module 365 may measure how much is not consumed (i.e., how much remains in a container) through image analysis of camera imaging, which may be used to determine how much was consumed.

In accordance with various embodiments, generally when humans consume liquids, each detected sip may correspond to more than one swallow. This may occur because most humans sip or otherwise intake more than they can swallow in one gulp. Thus, baselining may tend to be more accurate by using the number of detected swallows rather than a sip count, particularly considering the amount of liquid consumed with each swallow falls within a narrower range than the amount of liquid generally consumed with a sip. Thus, some embodiments may use a detected count of swallows for determining an amount consumed in consumption amount determination module 365. However, when a user is eating and drinking at the same time, counting sips may be a more accurate measure for fluid intake over counting swallows. Thus, in response to the processor determining that the user is drinking (as determined by the consumption type determination module 345), a swallowing count obtained from sensors and/or a data lookup may be used to determine the amount of liquid consumed by the user. Swallows may be detected based on data received from one or more electromyograms, pressure sensors, strain sensors, microphones, etc. In contrast, in response to the processor determining that the user is eating or drinking a liquid mixed with solids (e.g., ice or chunky soup), as may be determined by the consumption type determination module 345, a sip count obtained from sensors and/or a data lookup may be used to determine the amount of liquid consumed by the user. A sip count may be more accurate than a count of swallows in a mixed consumption case, if there is a good measure of how much is taken into the mouth on each sip, because there is less certainty about the amount of liquid the user is swallowing. Sips may be detected by camera images, as well as data from microphones, an electromyogram, motion sensors (e.g., on a smart necklace), etc. as well as information from other sensors that may confirms sips, such as IMU-based movement data, pressure sensors, strain sensors, etc. Also, in determining which counting method (i.e., counting sips versus counting swallows) is more appropriate, the processor may use the active and/or passive methods described above. Also, a baseline calibration routine may be provided that involves mixed consumption scenarios.

By way of a non-limiting example, sip and/or swallow count information may be received by an electronic device (e.g., user equipment 110) from sensors (e.g., the smart-glasses 130, sensor-equipped neck-strap 150, and smart-watch 170, etc.). The consumption amount determination module 365 may estimate a sip and/or swallow count based on sensor information by comparing such information to calibration information in a database, containing one or more data records, stored in local memory (e.g., 220, 258, 325) or from a remote source, such as a remote system (e.g., 315) or external resources (e.g., 320) using a transceiver (e.g., 256, 266) and related components.

The hydration level determination module 370 may be configured to estimate a hydration level of the user based on the determined amount of liquid consumed (as determined by the consumption amount determination module 365). In some embodiments, the estimation of the hydration level determined by the hydration level determination module 370 may be only a preliminary determination of hydration levels for the user based solely on a baseline consumption rate and a known or estimated volume of liquid consumed. Additional factors that affect hydration levels may be taken into account by the hydration level adjustment module 375 as described further below.

In some embodiments, the hydration level determination module 370 may be configured to track when hydration level checks are needed on behalf of the user. Hydration level checks may be needed at regular intervals or some other frequency deemed to be appropriate for a user. Also, the need for hydration level checks may be triggered by something the user has consumed, something the user has done or is doing, and/or external factors that may influence the user's hydration level.

Considering that 70-80% of total hydration comes from consumed liquids, even if the processor ignores liquid consumption that comes from food sources, a measure of hydration level based solely on liquid consumption may still determine the most significant driver of hydration in the user, namely liquid consumption. Thus, tracking only liquid consumption may provide a fairly accurate picture of total hydration, as well as for the preliminary determination of hydration levels calculated by the hydration level determination module 370.

Some embodiments may only measure the liquid consumed by a user, as opposed to the solids or solid/liquid mixes, but make adjustments that assume a percentage of liquid consumed from the non-measured consumables. For example, if only liquid consumption is being measured, which may only account for an estimated 80% of a user's liquid intake, the remaining percentage (i.e., 20% in this example) may be attributed to liquids consumed from foods (i.e., solids and solid/liquid mixes). Without actually measuring the solids or solid/liquid mixes consumed, various embodiment may assume the average percentage intake of liquid from those non-measured consumables that were consumed by the user. Thus, in this example, if only 80% of the user's hydration level is actually measured, the remaining 20% may be assumed to have been consumed. The amount of liquid consumed through solids or solid/liquid mixes may be driven by a user's diet, so other users who consume fewer fruits and/or vegetables (i.e., foods with higher hydration) may need more liquid intake from drinking fluids. Similarly, exercise and a user's regular exertion levels may control how much liquid intake that user needs. Also, a user's body composition may be considered when determining total hydration, since the more body fat makes up a user's body weight, the less water per pound (or kilogram) that user needs for optimal hydration.

By way of a non-limiting example, the volume of liquid that was consumed by the user may be received from the consumption amount determination module 365. Using body weight, the user's hydration level may be calculated as a percentage of that body's weight in fluids. In addition, using user bio-data, such as age, sex, body composition (e.g., body mass index), and previously determined and/or confirmed hydration level information, a processor may determine a more customized hydration level for the user.

The hydration level determination module 370 may additionally or alternatively obtain information from sensors that provide information to the electronic device 310 (e.g., user equipment 110) and/or local computing devices within the vicinity of the electronic device 310 (e.g., the smart-glasses 130, sensor-equipped neck-strap 150, and smart-watch 170, etc.). The hydration level determination module 370 may also receive sip and/or swallow count information by accessing a database, containing one or more data records, stored in local memory (e.g., 220, 258, 325) or from a remote source, such as a remote system (e.g., 315) or external resources (e.g., 320) using a transceiver (e.g., 256, 266) and related components. In addition, the hydration level determination module 370 may store the determined value(s) of the user's hydration level in local memory (e.g., 220, 258, 325) or that of a remote source, such as a remote system (e.g., 315) or external resources (e.g., 320) using a transceiver (e.g., 256, 266) and related components.

The hydration level adjustment module 375 may be configured to determine one or more adjustments to the preliminary hydration level determination (determined by the hydration level determination module 370) of a user. To determine all necessary adjustments, a processor may take into account all provided/available information. For example, the processor may have access to consumption information regarding the type of food and/or beverage consumed. Not all foods or liquids contribute to hydration in the same way. Certain liquids or foods can dehydrate the body, such as ones containing diuretics or antidiuretics. Gas sensors and camera images may be used by the electronic device to identify when these types of liquids and/or foods are consumed. Additionally, the water content of food may vary dramatically, thus the system may rely on manual user inputs to identify and track how much hydration is provided from foods. The processor may store or access from a database detailed hydration information about various liquids, foods, or combinations thereof. Some of that hydration information may have been manually entered by the user or obtained from other sources.

In some embodiments, the hydration level adjustment module 375 may make adjustments to the preliminarily determined hydration levels to account for the diuretics and antidiuretics. Gas sensors and camera images may be used by the system to identify the type of liquid consumed. The diuretics and antidiuretics quantities in liquids and food may vary dramatically, thus the system may rely on manual user inputs to identify and track how much of diuretics and antidiuretics are being consumed. The system may store common liquids and foods entered by the user, to make future entering of the diuretics and antidiuretics content of such liquids and foods easier if not automatic.

In some embodiments, the hydration level adjustment module 375 may make adjustments to the preliminarily determined hydration levels to account for a user's activity or exercise levels, as well as the user's health. For example, when a particular user consumes 80 ounces of liquid, the processor may determine a hydration level for that user. However, that same level of liquid consumption for the same user, when she has dramatically increased or decreased her daily level of exercise, may result in a different hydration level determination. Similarly, that same level of liquid consumption for a different user may result in a different hydration level determination because users processing of liquids and perspiration may vary (e.g., due to age, sex, body composition, health conditions, etc.). The hydration level adjustment module 375 may be used to make regular and/or periodic adjustments to a user's hydration level determinations, as well as accommodate different users.

By way of a non-limiting example, the processor (e.g., 210, 212, 214, 216, 218, 252, 260) of the electronic device may determine an adjustment to the hydration level determined by the hydration level determination module 370. In order to determine one or more adjustments to make to the preliminarily determined hydration level, the hydration level adjustment module 375 may receive information from sensors and/or access a database, containing one or more data records. The records may be stored in a local memory (e.g., 220, 258, 325) or received from a remote source, such as a remote system (e.g., 315) or external resources (e.g., 320) using a transceiver (e.g., 256, 266) and related components. The sensors may similarly be part of the electronic device or received from a remote source, such as a remote system (e.g., 315) or external resources (e.g., 320) using a transceiver (e.g., 256, 266) and related components. In addition, the hydration level determination module 370 may store the determined value(s) of the adjustment to the determined user's hydration level in local memory (e.g., 220, 258, 325) or that of a remote source, such as a remote system (e.g., 315) or external resources (e.g., 320) using a transceiver (e.g., 256, 266) and related components.

The hydration level reporting module 380 may be configured to report an indication of a determined hydration level based on the determined hydration level of the user. The reported indication of the determined hydration level may include an actual measurement of hydration level for the user, an indication of the percentage of the user's body weight in fluids, and/or a message indicating whether the user is dehydrated, over-hydrated, or within a normal range. The reported indication may be conveyed to the user via a display (e.g., 115), speaker, or a haptic feedback device on the electronic device (e.g., user equipment 110). Alternatively or additionally, the reported indication may be transmitted to a remote source, such as a remote system (e.g., 315) or external resources (e.g., 320) using a transceiver (e.g., 256, 266) and related components. For example, a processor may generate text or render an image on the display of the electronic device to provide an alert message when the user is dehydrated. Alternatively, the processor may transmit the alert message, which is configured to cause the remote computing device to perform the notification function on behalf of the electronic device.

By way of a non-limiting example, the processor (e.g., 210, 212, 214, 216, 218, 252, 260) of the electronic device may use the display (e.g., 115) to report the determined hydration level to the user. In addition, the processor (e.g., 210, 212, 214, 216, 218, 252, 260) of the electronic device may use one or more transceivers (e.g., 256, 266) to transmit the reported indication of the determined hydration level to a remote computing device (e.g., 120, 130, 140) with instructions regarding how, when, and/or under what circumstances the remote computing device notification functions should occur.

A remote computing device 315 may include one or more processors configured to execute computer program modules similar to those in the machine-readable instructions 335 described above. By way of a non-limiting examples, in addition to the wearable devices (e.g., 130, 150, 170) described above, the remote computing devices may include one or more of a smart-cup, a smart-straw, a smart-ring, a smart-appliance, a desktop computer, a laptop computer, a handheld computer, a tablet computing platform, a Netbook, another smart-phone, a gaming console, and/or other computing device.

External resources 320 may include remote servers storing a contacts database (or a backup copy of a contacts database), sources of information outside of system 300, external entities participating with system 300, and/or other resources. In some embodiments, some or all of the functionality attributed herein to external resources 320 may be provided by resources included in system 300.

The processor(s) 330 may be configured to execute modules 340, 345, 350, 355, 360, 365, 370, 375, and/or 380, and/or other modules. Processor(s) 330 may be configured to execute modules 340, 345, 350, 355, 360, 365, 370, 375, and/or 380, and/or other modules by software; hardware; firmware; some combination of software, hardware, and/or firmware; and/or other mechanisms for configuring processing capabilities on processor(s) 330. As used herein, the term "module" may refer to any component or set of components that perform the functionality attributed to the module. This may include one or more physical processors during execution of processor readable instructions, the processor readable instructions, circuitry, hardware, storage media, or any other components.

The description of the functionality provided by the different modules 340, 345, 350, 355, 360, 365, 370, 375, and/or 380 described below is for illustrative purposes, and is not intended to be limiting, as any of modules 340, 345, 350, 355, 360, 365, 370, 375, and/or 380 may provide more or less functionality than is described. For example, one or more of modules 340, 345, 350, 355, 360, 365, 370, 375, and/or 380 may be eliminated, and some or all of its functionality may be provided by other ones of modules 340, 345, 350, 355, 360, 365, 370, 375, and/or 380. As another example, processor(s) 330 may be configured to execute one or more additional modules that may perform some or all of the functionality attributed below to one of modules 340, 345, 350, 355, 360, 365, 370, 375, and/or 380.

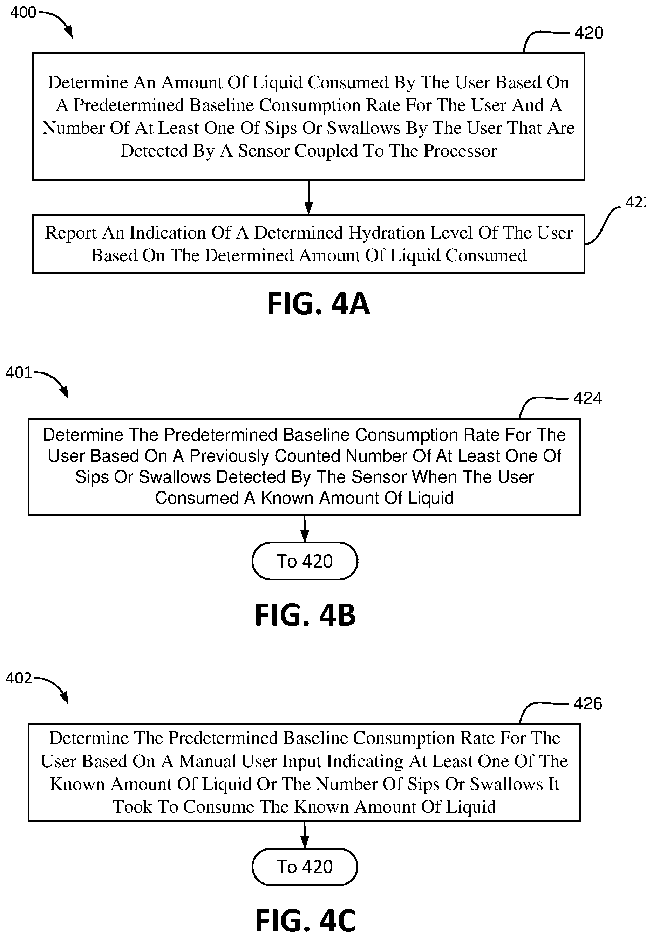

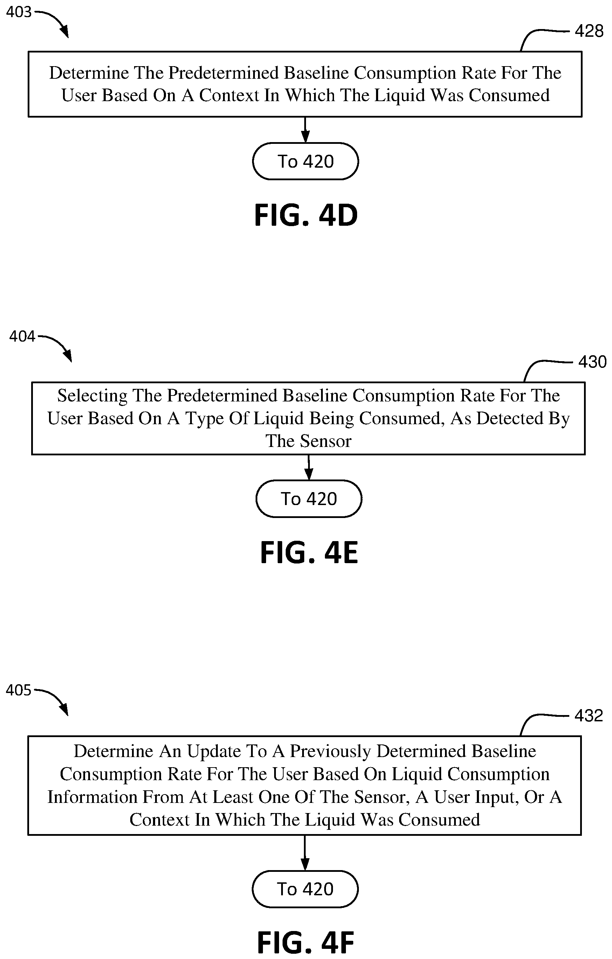

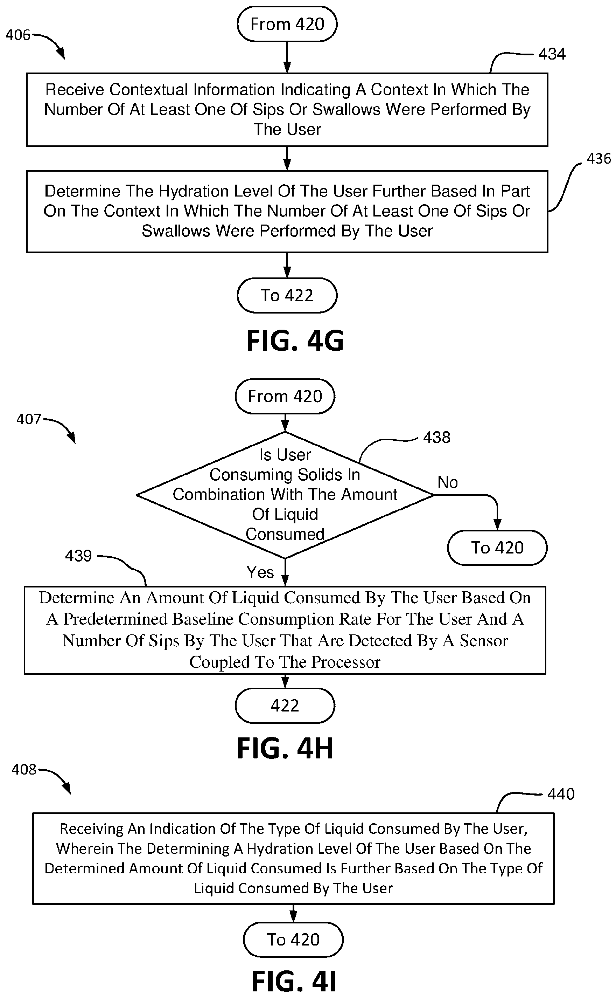

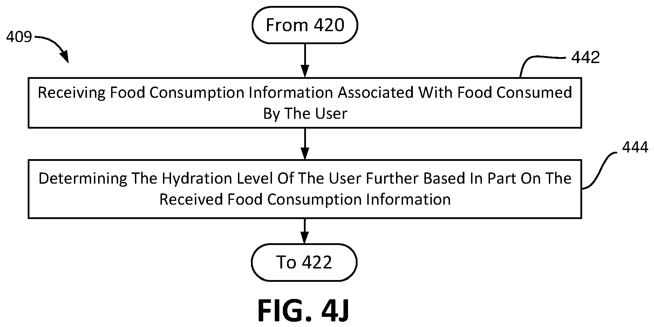

FIG. 4A illustrates a method 400 that may be executed by a processor of electronic device and/or one or more other computing devices for tracking hydration of a user in accordance with various embodiments. FIGS. 4B, 4C, 4D, 4E, 4F, 4G, 4H, 4I, and 4J illustrate additional or alternative operations in methods 401, 402, 403, 404, 405, 406, 407, 408, and 409 that may be performed as part of the method 400 in some embodiments. The operations of the methods 400, 401, 402, 403, 404, 405, 406, 407, 408, and 409 are intended to be illustrative. In some embodiments, methods 400, 401, 402, 403, 404, 405, 406, 407, 408, and 409 may be accomplished with one or more additional operations not described, and/or without one or more of the operations discussed. Additionally, the order in which the operations of the methods 400, 401, 402, 403, 404, 405, 406, 407, 408, and 409 are illustrated in FIGS. 4A, 4B, 4C, 4D, 4E, 4F, 4G, 4H, 4I, and 4J and described below is not intended to be limiting.

With reference to FIGS. 1-4I, the methods 400, 401, 402, 403, 404, 405, 406, 407, 408, and 409 may be implemented in one or more processors (e.g., 202, 204, 210, 212, 214, 216, 218, 252, 260) of electronic device (e.g., user equipment 110) and/or one or more other computing devices, including wearables (e.g., smart glasses 130) configured with processor-executable instructions stored on an non-transitory processor-readable storage medium. The one or more processors may include one or more devices configured through hardware, firmware, and/or software to be specifically designed for execution of one or more of the operations of the methods.

FIG. 4A illustrates a method 400 by which a processor of the electronic device may track hydration of a user in accordance with one or more embodiments.

In block 420, the processor of the electronic device may perform operations including determining an amount of liquid consumed by the user based on a predetermined baseline consumption rate for the user and a number of at least one of sips or swallows by the user that are detected by a sensor coupled to the processor. To make the determination in block 420, the processor may use the baseline consumption rate determination module (e.g., 355) and/or the baseline consumption rate adjustment module (e.g., 360) to determine the baseline consumption rate for the user, in conjunction with the consumption amount determination module (e.g., 365) to determine an amount of liquid consumed by the user. Also, in block 420, the processor may access a database, containing one or more data records, stored in local memory (e.g., 220, 258) or from a remote source, such as a remote system (e.g., 315) or external resources (e.g., 320) using a transceiver (e.g., 256, 266) and related components. The database may provide information regarding the baseline consumption rate of the user and/or the number of sips and/or swallows the user performed when consuming the liquid. Means for performing the operations of block 420 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

In block 422, the processor of the electronic device may perform operations including reporting an indication of a determined hydration level of the user based on the determined amount of liquid consumed. To perform the operations in block 422, the processor may use the hydration level determination module (e.g., 370), the hydration level adjustment module (e.g., 375), and/or the hydration level recording/reporting module (e.g., 380). Also, in block 422, the processor may cause a display (e.g., 115) to show an indication of the determined hydration level and/or show an alert if the determined hydration level is below a designated threshold (e.g., " ALERT, you have not consumed enough liquids today, drink some fluids. "). Alternatively, the display may show an amount of liquid consumed, combined with how much should have been consumed by that point in the day. Additionally, or as a further alternative, the display may show the user how much more the user should have consumed at that point in the day. Additionally, or as yet a further alternative, the processor, in block 422, may initiate an audible alert (e.g., sound or message), flash or flicker light, and/or produce a vibration or other haptic feedback to alert a user. Additionally or alternatively, the processor may instruct a remote computing device to display an indication of the determined hydration level, show an alert, initiate an audible alert (e.g., sound or message), flash or flicker light, and/or produce a vibration or other haptic feedback, if the determined hydration level is below a designated threshold. As a further alternative, the processor may initiate Means for performing the operations of block 422 may include a processor (e.g., 202, 204, 210, 212, 214, 216, 218, 252, 260) coupled to the display, a speaker, a vibration device, memory (e.g., 220, 258, 325) or from a remote source, such as a remote system (e.g., 315) or external resources (e.g., 320) using a transceiver (e.g., 256, 266) and related components.

Reporting an indication of the determined hydration level to the user may not only be helpful to inform the user, but may also provide a way to recalibrate the system. For example, if the processor reports a low hydration level that suggests the user is dehydrated, but the user has reason to believe he/she is not dehydrated (e.g., from light colored urine), the user may initiate a recalibration routine. This user feedback may be used to further train the hydration level determination module 370. In addition, such feedback may initiate an enhanced calibration routine, such as one that requests the user perform some measured consumptions (e.g., using wearables or other computing devices), a more direct test of dehydration (e.g., use a smart-toilet or otherwise collect a urine sample), or seek the aid of a third party (e.g., a medical practitioner).

Alternatively, if the processor reports a low hydration level and the user confirms he/she is dehydrated, such feedback is similarly useful.

FIG. 4B illustrates a method 401 in which the processor may determine the predetermined baseline consumption rate for the user based on a previously counted number of at least one of sips or swallows detected by the sensor when the user consumed a known amount of liquid. In block 424, the processor may determine the predetermined baseline consumption rate for the user based on a previously counted number of at least one of sips or swallows detected by the sensor when the user consumed a known amount of a known type of liquid during a calibration process. Different beverages and different conditions in which those beverages are consumed may establish different baseline consumption rates, which may be determined by the processor. For example, when drinking hot tea as part of the initial calibration process, the user may have performed, on average, thirty-six (36) swallows to finish drinking an eight (8) ounce cup of tea. Thus, thirty-six (36) swallows for eight (8) ounces of a hot liquid may be the predetermined baseline consumption rate determined by the processor. As another example, when drinking a strong alcoholic beverage, like whiskey straight up (i.e., no ice), the user may have performed, on average, twenty (20) sips to finish drinking two (2) ounces of whiskey. Thus, twenty (20) sips for two (2) ounces of a strong alcohol may be the predetermined baseline consumption rate determined by the processor. Similarly, when drinking a carbonated beverage, like seltzer, the user may have finished an eight (8) ounce can in thirty-six (36) sips, thereby establishing a baseline consumption rate for all or most carbonated drinks. In yet another example, when drinking a cool or room-temperature glass of water, the user may have performed, on average, fifteen (15) swallows to finish drinking a twelve (12) ounce glass. Thus, fifteen (15) swallows for twelve (12) ounces of water may be the predetermined baseline consumption rate determined by the processor.

To perform the operations in block 424, the processor may use the baseline consumption rate determination module 355 and/or the baseline consumption rate adjustment module 360. Means for performing the operations of block 424 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

Following the initial calibration operations in block 424, the processor may perform the operations in block 420 of the method 400 as described.

FIG. 4C illustrates a method 402 in which the processor may determine the predetermined baseline consumption rate for the user based on a manual user input indicating at least one of the known amount of liquid or the number of sips or swallows it took to consume the known amount of liquid. In block 426, the processor may determine the predetermined baseline consumption rate for the user based on a manual user input indicating at least one of the known amount of liquid or the number of sips or swallows it took the user to consume the known amount of liquid. For example, when drinking hot coffee as part of the initial calibration process, the user may have manually entered a baseline consumption rate of thirty-five (35) swallows to finish drinking a twelve (12) ounce mug of hot coffee. Thus, thirty-five (35) swallows for twelve (12) ounces of a hot liquid may be the predetermined baseline consumption rate determined by the processor. As another example, when consuming yogurt (i.e., a substance that is not entirely liquid and not entirely solid), the user may confirm a processor estimate of twelve (12) swallows to finish six (6) ounces of yogurt. Thus, twelve (12) swallows for six (6) ounces of yogurt may be the predetermined baseline consumption rate determined by the processor.

To perform the operations in block 426, the processor may use the baseline consumption rate determination module (e.g., 355) and/or the baseline consumption rate adjustment module (e.g., 360). Means for performing the operations of block 426 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

Following the initial calibration operations in block 426, the processor may perform the operations in block 420 of the method 400 as described.

FIG. 4D illustrates a method 403 in which the processor may determine the predetermined baseline consumption rate for the user based on a context in which the liquid was consumed. In block 428, the processor may determine the predetermined baseline consumption rate for the user based on the context in which the liquid was consumed. For example, the user may customarily drink a four (4) ounce energy drink at 6 am Monday through Friday, which the user consumes in two (2) gulps (i.e., two large swallows). To accommodate this, the user may perform the initial calibration procedure in the morning to determine a baseline consumption rate for liquid at that time of day. Thereafter, in response to the processor determining that the time is 6 am on a Tuesday, the processor may use a baseline consumption rate stored in memory that is associated with the customary energy drink. As another example, after vigorous aerobic exercise the user may drink water much more quickly than normal, taking only eight (8) swallows to finish an eight (8) ounce glass of water, as opposed to the twelve (12) swallows the user otherwise takes to finish that much water. Thus, in response to the processor determining that the user has just finished vigorous aerobic exercises, the processor may use a baseline consumption rate stored in memory following the initial calibration procedure of block 428, that is associated with the eight (8) swallows for eight (8) ounces of water.

To perform the operations in block 428, the processor may use the baseline consumption rate determination module (e.g., 355) and/or the baseline consumption rate adjustment module (e.g., 360). Means for performing the operations of block 428 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

Following the operations in block 428, the processor may perform the operations in block 420 of the method 400 as described.

FIG. 4E illustrates a method 404 in which the processor selects the predetermined baseline consumption rate for the user based on a type of liquid being consumed, as detected by the sensor. In block 430, the processor may use sensor input received by the processor that indicates what type of fluid is being consumed to select the predetermined baseline consumption rate for the user based on the context in which the liquid was consumed. For example, the user may be drinking hot tea, which a thermal sensor on the user's smart-glasses has detected to be a hot-type beverage (i.e., above a predetermined temperature). The processor may receive the indication of the hot-type beverage being consumed from the thermal sensor and, based on that information, select the predetermined baseline consumption rate determined for hot-type beverages. To determine the predetermined baseline consumption rate for tea or other hot-type beverages, the user may perform the initial calibration procedure drinking the same or similar hot tea and/or some other hot-type beverage under controlled and measured conditions. Thereafter, in response to the processor determining that the user is drinking the same or similar hot-type beverage, the processor may use a baseline consumption rate stored in memory that is associated with that type of liquid. Thus, in response to the processor receiving an indication of the type of liquid being consumed, the processor selects an appropriate baseline consumption rate stored in memory for determining the amount of liquid consumed by the user.

To perform the operations in block 430, the processor may use the consumption type determination module (e.g., 345) and/or the baseline consumption rate determination module (e.g., 355). Means for performing the operations of block 430 may include a processor (e.g., 202, 204, 210, 212, 214, 216, 218, 252, 260) coupled to memory (e.g., 220, 258, 325), and one or more devices with sensors (e.g., the smart-glasses 130, sensor-equipped neck-strap 150, smart-watch 170, ear-piece 180, etc.), or from a remote source, such as a remote system (e.g., 315) or external resources (e.g., 320) using a transceiver (e.g., 256, 266) and related components.

Following the operations in block 430, the processor may perform the operations in block 420 of the method 400 as described.

FIG. 4F illustrates a method 405 in which the processor may determine an update to a previously determined baseline consumption rate for the user based on liquid consumption information from at least one of the sensor, a user input, or a context in which the liquid was consumed. In block 432, the processor may determine an update to a previously determined baseline consumption rate for the user based on liquid consumption information from at least one of a sensor, a user input, or a context in which the liquid was consumed. For example, the user may use a smart-cup several days in a row to repeatedly drink ten (10) ounces of cool tea, which a sensor-equipped neck-strap (e.g., 150) detects takes the user thirteen (13) swallows to consume. As part of the operations in block 432, the processor may compare a previously determined baseline consumption rate for the user drinking cool tea to most recent series of smart-cup usage to determine that the previously determined baseline consumption rate is not accurate and needs updating. As another example, the user may manually input an indication that a previously determined baseline consumption rate needs update, which initiates a calibration protocol. In response to the processor determining that the previously determined baseline consumption rate needs update, the processor may determine an update to the previously determined baseline consumption rate in block 432. In a further example, when an ambient temperature drops below freezing, records may indicate the user's baseline consumption rate also drops ten percent (10%) from its usual rate. Thus, in response to the processor determining that the ambient temperature is below freezing, the processor may identify and/or compile an update to the previously determined baseline consumption rate for drinking in freezing conditions.

To perform the operations in block 432, the processor may use the baseline consumption rate determination module (e.g., 355) and/or the baseline consumption rate adjustment module (e.g., 360). Means for performing the operations of block 432 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

Following the operations in block 432, the processor may perform the operations in block 420 of the method 400 as described, wherein the predetermined baseline consumption rate is based on the update to the previously determined baseline consumption rate for the user.

FIG. 4G illustrates a method 406 by which a processor of the electronic device may receive contextual information and determine the hydration level of the user further based in part on the context in which the number of at least one of sips or swallows were performed by the user in accordance with some embodiments.

In block 434, following the operations in block 420, the processor of the electronic device may perform operations including receiving contextual information indicating a context in which the number of at least one of sips or swallows were performed by the user. To perform the operations in block 434, the processor may use the context information receiving module 350. Means for performing the operations of block 434 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

By way of a non-limiting example, contextual information may be received from sensors that provide information to the electronic device (e.g., user equipment 110) and/or local computing devices within the vicinity of the electronic device (e.g., the smart-glasses 130, sensor-equipped neck-strap 150, smart-watch 170, ear-piece 180, etc.). The contextual information may also be received by accessing a database, containing one or more data records, stored in local memory (e.g., 220, 258, 325) or from a remote source, such as a remote system (e.g., 315) or external resources (e.g., 320) using a transceiver (e.g., 256, 266) and related components.

In block 436, the processor may determine the hydration level of the user further based in part on the context in which the number of at least one of sips or swallows were performed by the user. For example, the processor may receive an indication that the user has just been performing a high intensity exercise. A database lookup may indicate that such activity requires ten percent (10%) more liquid intake to maintain proper hydration levels. Thus, based on the contextual information about the user's activity level, the processor may determine the user's hydration level, while taking into account the added exertion. As another example, the processor may detect that the user has been consuming a drink and/or food that has anti-diuretic properties. A database lookup may indicate that such anti-diuretics require five percent (5%) less liquid intake to maintain proper hydration levels, due to the water retention properties of the anti-diuretic. Thus, based on the contextual information about the user's drink and/or food consumption, the processor may determine the user's hydration level, while taking into account the contextual information.

To perform the operations in block 436, the processor may use the hydration level determination module 370 and/or the hydration level adjustment module 375 along with the contextual information receiving module 350. Also, in block 436, the processor may access a database, containing one or more data records, stored in local memory (e.g., 220, 258) or from a remote source, such as a remote system (e.g., 315) or external resources (e.g., 320) using a transceiver (e.g., 256, 266) and related components. The database may provide information for determining the hydration level of the user, including the baseline consumption rate of the user and/or the number of sips and/or swallows the user performed when consuming the liquid. In addition, the database may include contextual information relevant to the calculation of the user's hydration level. Means for performing the operations of block 436 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

Following the operations in block 436, the processor may perform the operations in block 422 of the method 400 as described.

FIG. 4H illustrates a method 407 in which the processor may determine whether the user is consuming solids in combination with the amount of liquid consumed in order to determine whether to measure liquid consumption using sips rather than swallows.

In determination block 438, following the operations in block 420, the processor of the electronic device may perform operations including determining whether the user is consuming solids in combination with the amount of liquid being consumed. For example, the user may have a drink with ice or be eating a chunky soup (i.e., with large chunks of meat, vegetables, and/or noodles). To make the determination in determination block 438, the processor may use the consumption type determination module (e.g., 345). Also, in determination block 438, the processor may access a database, containing one or more data records, stored in local memory (e.g., 220, 258) or from a remote source, such as a remote system (e.g., 315) or external resources (e.g., 320) using a transceiver (e.g., 256, 266) and related components. The database may provide information about the substance being consumed. Means for performing the operations of block 420 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

In response to determining the user is not consuming solids in combination with the amount of liquid consumed (i.e., determination block 438 = "No"), the processor may perform the operations in block 420.

In response to determining the user is consuming solids in combination with the amount of liquid consumed (i.e., determination block 438 = "Yes"), the processor may perform operations including determining an amount of liquid consumed by the user based on a predetermined baseline consumption rate for the user and a number of sips by the user that are detected by a sensor coupled to the processor in block 439. To make the determination in block 439, the processor may use the baseline consumption rate determination module (e.g., 355) and/or the baseline consumption rate adjustment module (e.g., 360), to determine the baseline consumption rate for the user, in conjunction with the consumption amount determination module (e.g., 365) to determine an amount of liquid consumed by the user. Also in block 439, the processor may access a database, containing one or more data records, stored in local memory (e.g., 220, 258) or from a remote source, such as a remote system (e.g., 315) or external resources (e.g., 320) using a transceiver (e.g., 256, 266) and related components. The database may provide information as to the baseline consumption rate of the user and/or the number of sips and/or swallows the user performed when consuming the liquid. Means for performing the operations of block 439 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

Following the operations in block 439, the processor may perform the operations in block 422 of the method 400 as described.

FIG. 4I illustrates a method 408 by which a processor of the electronic device receives an indication of the type of liquid consumed by the user, in accordance with some embodiments.

In block 440, the processor of the electronic device performs operations including receiving an indication of the type of liquid consumed by the user. To perform the operations in block 440, the processor may use the consumption type determination module 345. Means for performing the operations of block 440 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

By way of a non-limiting example, an indication of the type of liquid consumed by the user may be received from sensors that provide information to the electronic device (e.g., user equipment 110) and/or local computing devices within the vicinity of the electronic device (e.g., the smart-glasses 130, sensor-equipped neck-strap 150, smart-watch 170, ear-piece 180, etc.). The indication of the type of liquid consumed may also be received by accessing a database, containing one or more data records, stored in local memory (e.g., 220, 258, 325) or from a remote source, such as a remote system (e.g., 315) or external resources (e.g., 320) using a transceiver (e.g., 256, 266) and related components.

Following the operations in block 440, the processor may perform the operations in block 420 of the method 400 as described in which the determined amount of liquid consumed by the user is further based on the type of liquid consumed by the user.

FIG. 4J illustrates a method 409 by which a processor of the electronic device may receive food consumption information and determine the hydration level of the user further based in part on the received food consumption information in accordance with some embodiments.

In block 442, following the operations in block 420, the processor of the electronic device may perform operations including receiving food consumption information associated with food consumed by the user. For example, the processor may receive liquid content information about food or interactive properties of foods that include diuretics, anti-diuretics, and/or other properties. To perform the operations in block 442, the processor may use the consumption type determination module 345. Means for performing the operations of block 442 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

By way of a non-limiting example, consumption information may be received from sensors that provide information to the electronic device (e.g., user equipment 110) and/or local computing devices within the vicinity of the electronic device (e.g., the smart-glasses 130, sensor-equipped neck-strap 150, smart-watch 170, ear-piece 180, etc.). The consumption information may also be received by accessing a database, containing one or more data records, stored in local memory (e.g., 220, 258, 325) or from a remote source, such as a remote system (e.g., 315) or external resources (e.g., 320) using a transceiver (e.g., 256, 266) and related components.

In block 444, the processor may determine the hydration level of the user further based in part on the received food consumption information. For example, the processor may receive an indication that the user has just consumed a large piece of watermelon, some Jell-O^{®}, or other food. A database lookup may indicate that such food includes a certain percentage of liquid on average. Thus, based on the received food consumption information, the processor may determine an estimation of the user's hydration level.

To perform the operations in block 444, the processor may use the hydration level determination module 370 and/or the hydration level adjustment module 375 along with the consumption type determination module 345. Also, in block 444, the processor may access a database, containing one or more data records, stored in local memory (e.g., 220, 258) or from a remote source, such as a remote system (e.g., 315) or external resources (e.g., 320) using a transceiver (e.g., 256, 266) and related components. The database may provide information for determining the hydration level of the user, including the liquid content of the identified food that was consumed. In addition, the database may include consumption information relevant to the calculation of the user's hydration level. Means for performing the operations of block 436 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

Following the operations in block 444, the processor may perform the operations in block 422 of the method 400 as described.

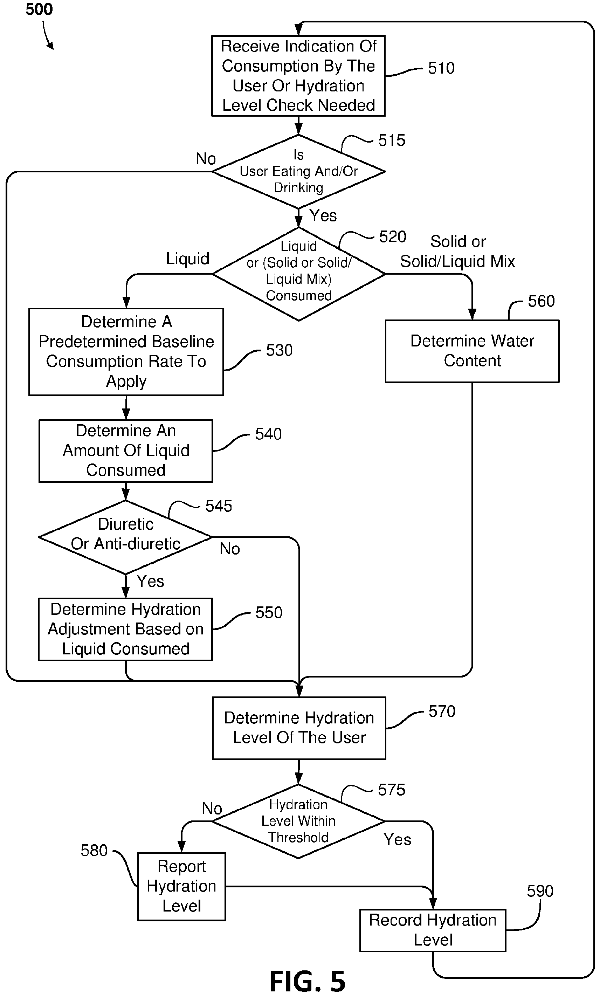

FIG. 5 illustrates a method 500 that may be executed by a processor of electronic device and/or one or more other computing devices for tracking hydration of a user in accordance with various embodiments. The operations of the method 500 are intended to be illustrative. In some embodiments, method 500 may be accomplished with one or more additional operations not described, and/or without one or more of the operations discussed. Additionally, the order in which the operations of the method 500 are illustrated in FIG. 5 and described below is not intended to be limiting.

With reference to FIGS. 1-5, the method 500 may be implemented in one or more processors (e.g., 202, 204, 210, 212, 214, 216, 218, 252, 260) of electronic device (e.g., user equipment 110), wearables (e.g., 130, 150, 170), and other computing devices configured with processor-executable instructions stored on an non-transitory processor-readable storage medium. The one or more processors may include one or more devices configured through hardware, firmware, and/or software to be specifically designed for execution of one or more of the operations of the methods.

In block 510, the processor of the electronic device may perform operations including receiving an indication of consumption by the user or determining that a hydration level check is needed. The received indication of consumption may be from a sensor input consistent with consumption, such as swallowing, sipping, chewing, etc. The determination that the hydration level check is needed may be based on various factors, such as the time, an interval since the last check, the user's activity, contextual information, etc. To perform the operations in block 510, the processor may use the consumption indication receiving module 340, such as a clock. Means for performing the operations of block 510 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

By way of a non-limiting example, consumption indication information may be received from sensors that provide information to the electronic device (e.g., user equipment 110) and/or local computing devices within the vicinity of the electronic device (e.g., the smart-glasses 130, sensor-equipped neck-strap 150, smart-watch 170, ear-piece 180, etc.). The consumption indication information may also be received by accessing a database, containing one or more data records, stored in local memory (e.g., 220, 258, 325) or from a remote source, such as a remote system (e.g., 315) or external resources (e.g., 320) using a transceiver (e.g., 256, 266) and related components.

In determination block 515, the processor may determine whether the user is eating and/or drinking based on the indication of consumption received in block 510. To make the determination in determination block 515, the processor may use the consumption type determination module (e.g., 345). Also, in determination block 515, the processor may access a database, containing one or more data records, stored in local memory (e.g., 220, 258) or from a remote source, such as a remote system (e.g., 315) or external resources (e.g., 320) using a transceiver (e.g., 256, 266) and related components. The database may provide information about the substance being consumed. Means for performing the operations of block 515 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

In response to determining that the user is not eating and/or drinking something (i.e., determination block 515 = "No"), the processor may determine a hydration level of the user in block 570.

In response to determining that the user is eating and/or drinking something (i.e., determination block 515 = "Yes"), the processor may determine whether the substance being consumed is a liquid or whether the substance being consumed is either a solid or solid/liquid mix. To make the determination in determination block 520, the processor may use the consumption type determination module (e.g., 345). Also, in determination block 520, the processor may access a database, containing one or more data records, stored in local memory (e.g., 220, 258) or from a remote source, such as a remote system (e.g., 315) or external resources (e.g., 320) using a transceiver (e.g., 256, 266) and related components. The database may provide information about the substance being consumed. Means for performing the operations of block 520 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

In response to determining that the user is drinking a liquid (i.e., determination block 520 = "Liquid"), the processor may determine a predetermined baseline consumption rate to apply for the user to the liquid consumption. Various factors may be taken into account for determining which predetermined baseline consumption rate to use, such as whether a known liquid is being consumed, whether a user input is received or available, and/or whether contextual information is available or applicable. The determination of the predetermined baseline consumption rate in block 530 may include the operations in blocks 424, 426, 428, and/or 430 of the methods 401, 402, 403, and/or 404 as described.

In block 540, the processor may determine an amount of liquid consumed by the user by counting the number of sips or swallows and correlating that count to total liquid consumed based on the predetermined baseline consumption rate, such as by using the operations in blocks 420 or 439 of the methods 400 or 406 as described.

In determination block 545, the processor may determine whether the substance being consumed includes a diuretic or anti-diuretic. To make the memory (e.g., 220, 258) or from a remote source, such as a remote system (e.g., 315) or external resources (e.g., 320) using a transceiver (e.g., 256, 266) and related components. The database may provide information about the diuretic or anti-diuretic nature of the substance being consumed. Means for performing the operations of block 520 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

In response to determining that the substance being consumed includes a diuretic or anti-diuretic (i.e., determination block 545 = "Yes"), the processor may determine a hydration adjustment based on the liquid consumed in block 550. To perform the operations in block 550, the processor may use the hydration level adjustment module 375. Also, in block 550, the processor may access a database, containing one or more data records, stored in local memory (e.g., 220, 258) or from a remote source, such as a remote system (e.g., 315) or external resources (e.g., 320) using a transceiver (e.g., 256, 266) and related components. The database may provide information for determining an adjustment to the hydration level of the user based on the type and amount of diuretic or anti-diuretic consumed. Means for performing the operations of block 550 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

In response to determining the user is eating a solid or solid/liquid mix (i.e., determination block 520 = "(Solid or Solid/Liquid Mix)"), the processor may determine a water content of the solid or solid/liquid mix that was consumed in block 560. In determining the water content, the processor may use the consumption type determination module 345, which may access a database containing one or more data records of information regarding water content in various foods and beverages, stored in memory (e.g., 220, 258, 325) or from a remote source, such as a remote system (e.g., 315) or external resources (e.g., 320) using a transceiver (e.g., 256, 266) and related components. The processor of the electronic device may access the data records to determine how much water is in the solid or solid/liquid mix that was consumed. For example, a lookup table may provide an estimate of how much water is contained in particular foods or a beverage with solids (e.g., fruit, ice, etc.) therein.

In response to determining that the substance being consumed does not include a diuretic or anti-diuretic (i.e., determination block 545 = "No"), after determining the user is not eating and/or drinking anything (i.e., determination block 515 = "No"), the hydration adjustment in block 550, or after determining the water content of the solid or solid/liquid mix in block 560, the processor may determine a hydration level of the user in block 570. The processor may determine the hydration level of the user based on the liquid consumed and optionally added water from any food that was consumed. To perform the operations in block 444, the processor may use the hydration level determination module 370 and/or the hydration level adjustment module 375 along with the consumption type determination module 345. Also, in block 444, the processor may access a database, containing one or more data records, stored in local memory (e.g., 220, 258) or from a remote source, such as a remote system (e.g., 315) or external resources (e.g., 320) using a transceiver (e.g., 256, 266) and related components. The database may provide information for determining the hydration level of the user, including the liquid content of identified foods that were consumed. In addition, the database may include consumption information relevant to the calculation of the user's hydration level. Means for performing the operations of block 436 may include a processor (e.g., 202, 204, 210, 212, 214, 216, 218, 252, 260) coupled to memory (e.g., 220, 258, 325) or from a remote source, such as a remote system (e.g., 315) or external resources (e.g., 320) using a transceiver (e.g., 256, 266) and related components.

In determination block 575, the processor may determine whether the determined hydration level is within a threshold. For example, the threshold may be a minimum healthy hydration level. As another example the threshold may be a target healthy threshold for a particular activity level of the user. As another example, the threshold may be in the form of a minimum threshold and a maximum threshold, in which the minimum threshold is a minimum healthy level of hydration and the maximum threshold is a maximum level of hydration consistent with healthy activities. Further, the threshold may be user specific, such as based upon the age, weight, sex, medical condition, and other personal health characteristics of the user.

In response to determining that the determined hydration level is not within the threshold (i.e., determination block 575 = "No"), the processor may alert the user, such as by displaying a report of the hydration level in block 580, such as illustrated in FIG. 1. In block 580, the processor reporting the hydration level may be performed similar to the operations in block 422 of the method 400 as described.

In response to determining that the determined hydration level is within the threshold (i.e., determination block 575 = "Yes") or after reporting the hydration level in block 580, the processor may record the hydration level in local memory (e.g., 220, 258) or in memory of a remote source, such as a remote system (e.g., 315) or external resources (e.g., 320) using a transceiver (e.g., 256, 266) and related components, in block 590.

The operations in the method 500 may be performed whenever the user begins consuming liquids or food.

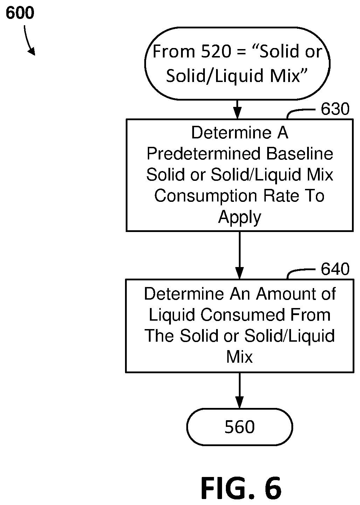

FIG. 6 illustrates a method 600 that may be performed as part of the method 500 by using separate baseline consumption rates for liquids and foods (i.e., solids or solid/liquid mixes in some embodiments). The operations in the method 600 may be executed by a processor of electronic device and/or one or more other computing devices for tracking hydration of a user in accordance with various embodiments. The operations of the method 600 are intended to be illustrative. In some embodiments, method 600 may be accomplished with one or more additional operations not described, and/or without one or more of the operations discussed. Additionally, the order in which the operations of the method 600 are illustrated in FIG. 6 and described below is not intended to be limiting.

With reference to FIGS. 1-6, the method 600 may be implemented in one or more processors (e.g., 202, 204, 210, 212, 214, 216, 218, 252, 260) of electronic device (e.g., user equipment 110), wearables (e.g., 130, 150, 170), and other computing devices configured with processor-executable instructions stored on an non-transitory processor-readable storage medium of. The one or more processors may include one or more devices configured through hardware, firmware, and/or software to be specifically designed for execution of one or more of the operations of the methods.

In response to determining that the user is consuming or has consumed a solid or solid/liquid mix (i.e., determination block 520 = "Solid or Solid/Liquid Mix"), the processor may determine a predetermined baseline solid or solid/liquid mix consumption rate to apply for the user to the solid or solid/liquid mix consumption in block 630. The determination of the predetermined baseline solid or solid/liquid mix consumption rate in block 630 may be performed using operations similar to those applied to determinations of liquid consumption in blocks 424, 426, 428 and/or 530 of the methods 401, 402, 403 and/or 500 as described.

In block 640, the processor may determine an amount of liquid consumed from the solid or solid/liquid mix that was consumed by the user. The determination of the amount of liquid consumed from the solid or solid/liquid mix consumed in block 640 may be performed using operations similar to those applied in blocks 420 or 439 of the methods 400 or 406 as described.

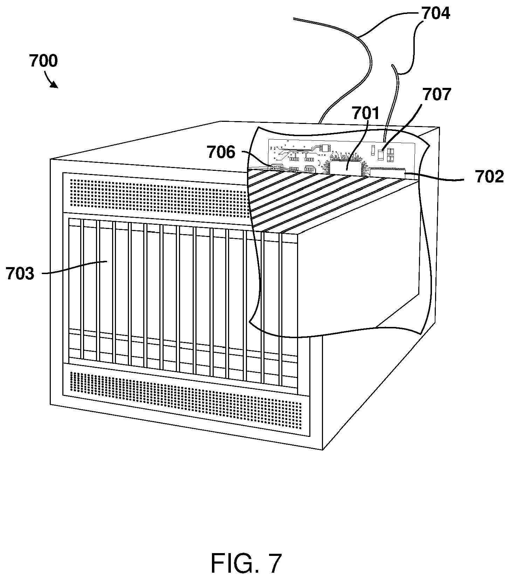

The various embodiments (including, but not limited to, embodiments discussed above with reference to FIGS. 1-6) may be implemented on a variety of computing devices, an example of which is illustrated in FIG. 7 in the form of a server. With reference to FIGS. 1-7, the network computing device 700 may include a processor 701 coupled to volatile memory 702 and a large capacity nonvolatile memory, such as a disk drive 703. The network computing device 700 may also include a peripheral memory access device such as a floppy disc drive, compact disc (CD) or digital video disc (DVD) drive 706 coupled to the processor 701. The network computing device 700 may also include network access ports 704 (or interfaces) coupled to the processor 701 for establishing data connections with a network, such as the Internet and/or a local area network coupled to other system computers and servers. The network computing device 700 may include one or more antennas 707 for sending and receiving electromagnetic radiation that may be connected to a wireless communication link. The network computing device 700 may include additional access ports, such as USB, Firewire, Thunderbolt, and the like for coupling to peripherals, external memory, or other devices.

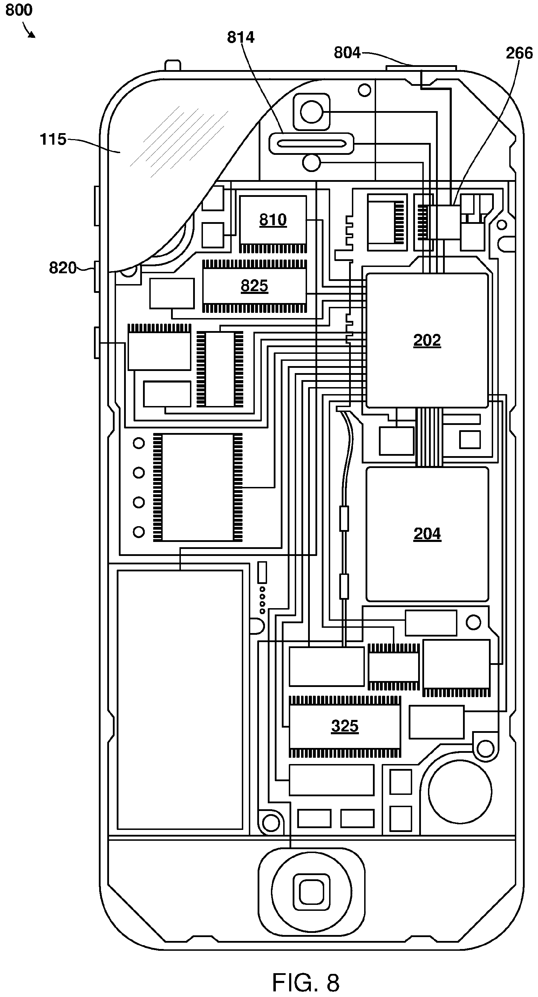

The various embodiments (including, but not limited to, embodiments discussed above with reference to FIGS. 1-6) may be implemented on a variety of computing devices, an example of which is illustrated in FIG. 8 in the form of a mobile computing device. With reference to FIGS. 1-8, a mobile computing device 800 may include a first SoC 202 (e.g., a SoC-CPU) coupled to a second SoC 204 (e.g., a 5G capable SoC), such as D2D links establish in the dedicated ITS 5.9 GHz spectrum communications). The first and/or second SOCs 202, 204 may be coupled to internal memory 325, 825, a display 115, and to a speaker 814. Additionally, the mobile computing device 800 may include one or more antenna 804 for sending and receiving electromagnetic radiation that may be connected to one or more wireless transceivers 266 (e.g., a wireless data link and/or cellular transceiver, etc.) coupled to one or more processors in the first and/or second SOCs 202, 204. Mobile computing devices 800 may also include menu selection buttons or rocker switches 820 for receiving user inputs.

Mobile computing devices 800 may additionally include a sound encoding/decoding (CODEC) circuit 810, which digitizes sound received from a microphone into data packets suitable for wireless transmission and decodes received sound data packets to generate analog signals that are provided to the speaker to generate sound. Also, one or more of the processors in the first and/or second SOCs 202, 204, wireless transceiver 266 and CODEC circuit 810 may include a digital signal processor (DSP) circuit (not shown separately).

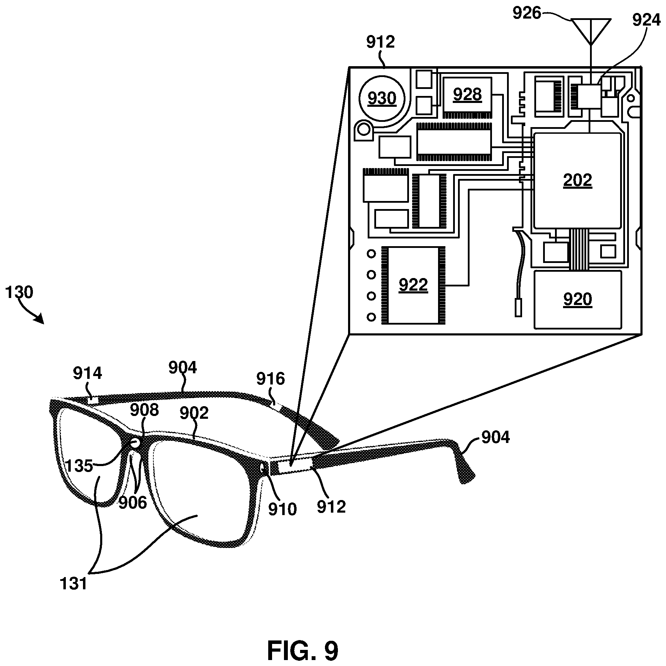

Various embodiments (including embodiments discussed above with reference to FIGS. 1-6) may be implemented on a variety of wearable devices, an example of which is illustrated in FIG. 9 in the form of a pair of smart glasses 130. With reference to FIGS. 1-9, a pair of smart glasses 130 may operate like conventional eye glasses, but with enhanced computer features and sensors, like a built-in camera 135 and heads-up display or augmented reality features on or near the lenses 131. Like any glasses, smart glasses may include a frame 902 coupled to temples 904 that fit alongside the head and behind the ears of a wearer. The frame 902 holds the lenses 131 in place before the wearer's eyes when nose pads 906 on the bridge 908 rest on the wearer's nose.

In some embodiments, smart-glasses 130 may include an image rendering device 914 (e.g., an image projector), which may be embedded in one or both temples 904 of the frame 902 and configured to project images onto the optical lenses 131. In some embodiments, the image rendering device 914 may include a light-emitting diode (LED) module, a light tunnel, a homogenizing lens, an optical display, a fold mirror, or other components well known projectors or head-mounted displays. In some embodiments (e.g., those in which the image rendering device 914 is not included or used), the optical lenses 131 may be, or may include, see-through or partially see-through electronic displays. In some embodiments, the optical lenses 131 include image-producing elements, such as see-through Organic Light-Emitting Diode (OLED) display elements or liquid crystal on silicon (LCOS) display elements. In some embodiments, the optical lenses 131 may include independent left-eye and right-eye display elements. In some embodiments, the optical lenses 131 may include or operate as a light guide for delivering light from the display elements to the eyes of a wearer.

The smart-glasses 130 may include a number of external sensors that may be configured to obtain information about wearer actions and external conditions that may be useful for sensing images, sounds, muscle motions and other phenomenon that may be useful for detecting when the wearer is consuming liquids as described. In some embodiments, smart-glasses 130 may include a camera 135 configured to image objects in front of the wearer in still images or a video stream, which may be transmitted to another computing device (e.g., a mobile device 800) for analysis. In some embodiments, smart-glasses 130 may include a microphone 910 positioned and configured to record sounds in the vicinity of the wearer. In some embodiments, multiple microphones may be positioned in different locations on the frame 902, such as on a distal end of the temples 904 near the jaw, to record sounds emanating from the wearer, such as jaw movements, chewing sounds, swallowing sounds, and the like. In some embodiments, smart-glasses 130 may include one or more electromyograms 916 mounted on one or both temples 904, such as near the temples or above the ears, and configured to measure electrical activity of the nerves and muscles in the jaw and temple area of a wearer. In some embodiments, smart-glasses 130 may include pressure sensors, such on the nose pads 906, configured to sense facial movements. In some embodiments, smart glasses 130 may include other sensors (e.g., a thermometer, heart rate monitor, body temperature sensor, pulse oximeter etc.) for collecting information pertaining to environment and/or user conditions that may be useful for detecting when the wearer is consuming liquids.

The processing system 912 may include a processing and communication SOC 202, which may include one or more processors (e.g., 202, 204, 210, 212, 214, 216, 218), one or more of which may be configured with processor-executable instructions to perform operations of various embodiments. The processing and communication SOC 202 may be coupled to internal sensors 920, internal memory 922, and communication circuitry 924 coupled one or more antenna 926 for establishing a wireless data link with an external computing device (e.g., a mobile device 800), such as via a Bluetooth link. The processing and communication SOC 202 may also be coupled to sensor interface circuitry 928 configured to control and received data from a camera 135, microphone(s) 910, one or more electromyograms 916, and other sensors positioned on the frame 902.

The internal sensors 920 may include an IMU that includes electronic gyroscopes, accelerometers, and a magnetic compass configured to measure movements and orientation of the wearer's head. The internal sensors 920 may further include a magnetometer, an altimeter, an odometer, and an atmospheric pressure sensor, as well as other sensors useful for determining the orientation and motions of the smart glasses 130. Such sensors may be useful in various embodiments for detecting head motions associated with consuming liquids as described.

The processing system 912 may further include a power source such as a rechargeable battery 930 coupled to the SOC 202 as well as the external sensors on the frame 902.

The processors implementing various embodiments may be any programmable microprocessor, microcomputer or multiple processor chip or chips that can be configured by software instructions (applications) to perform a variety of functions, including the functions of the various aspects described in this application. In some communication devices, multiple processors may be provided, such as one processor dedicated to wireless communication functions and one processor dedicated to running other applications. Typically, software applications may be stored in the internal memory before they are accessed and loaded into the processor. The processor may include internal memory sufficient to store the application software instructions.

As used in this application, the terms "component," "module," "system," and the like are intended to include a computer-related entity, such as, but not limited to, hardware, firmware, a combination of hardware and software, software, or software in execution, which are configured to perform particular operations or functions. For example, a component may be, but is not limited to, a process running on a processor, a processor, an object, an executable, a thread of execution, a program, and/or a computer. By way of illustration, both an application running on a processor of a communication device and the communication device may be referred to as a component. One or more components may reside within a process and/or thread of execution and a component may be localized on one processor or core and/or distributed between two or more processors or cores. In addition, these components may execute from various non-transitory computer readable media having various instructions and/or data structures stored thereon. Components may communicate by way of local and/or remote processes, function or procedure calls, electronic signals, data packets, memory read/writes, and other known network, computer, processor, and/or process related communication methodologies.

A number of different cellular and mobile communication services and standards are available or contemplated in the future, all of which may implement and benefit from the various aspects. Such services and standards may include, e.g., third generation partnership project (3GPP), long term evolution (LTE) systems, third generation wireless mobile communication technology (3G), fourth generation wireless mobile communication technology (4G), fifth generation wireless mobile communication technology (5G), global system for mobile communications (GSM), universal mobile telecommunications system (UMTS), 3GSM, general packet radio service (GPRS), code division multiple access (CDMA) systems (e.g., cdmaOne, CDMA1020TM), EDGE, advanced mobile phone system (AMPS), digital AMPS (IS-136/TDMA), evolution-data optimized (EV-DO), digital enhanced cordless telecommunications (DECT), Worldwide Interoperability for Microwave Access (WiMAX), wireless local area network (WLAN), Wi-Fi Protected Access I & II (WPA, WPA2), integrated digital enhanced network (iden), C-V2X, V2V, V2P, V2I, and V2N, etc. Each of these technologies involves, for example, the transmission and reception of voice, data, signaling, and/or content messages. It should be understood that any references to terminology and/or technical details related to an individual telecommunication standard or technology are for illustrative purposes only, and are not intended to limit the scope of the claims to a particular communication system or technology unless specifically recited in the claim language.

Various aspects illustrated and described are provided merely as examples to illustrate various features of the claims. However, features shown and described with respect to any given aspect are not necessarily limited to the associated aspect and may be used or combined with other aspects that are shown and described. Further, the claims are not intended to be limited by any one example aspect. For example, one or more of the operations of the methods may be substituted for or combined with one or more operations of the methods.

The foregoing method descriptions and the process flow diagrams are provided merely as illustrative examples and are not intended to require or imply that the operations of various aspects must be performed in the order presented. As will be appreciated by one of skill in the art the order of operations in the foregoing aspects may be performed in any order. Words such as "thereafter," "then," "next," etc. are not intended to limit the order of the operations; these words are used to guide the reader through the description of the methods. Further, any reference to claim elements in the singular, for example, using the articles "a," "an," or "the" is not to be construed as limiting the element to the singular.

Various illustrative logical blocks, modules, components, circuits, and algorithm operations described in connection with the aspects disclosed herein may be implemented as electronic hardware, computer software, or combinations of both. To clearly illustrate this interchangeability of hardware and software, various illustrative components, blocks, modules, circuits, and operations have been described above generally in terms of their functionality. Whether such functionality is implemented as hardware or software depends upon the particular application and design constraints imposed on the overall system. Skilled artisans may implement the described functionality in varying ways for each particular application, but such aspect decisions should not be interpreted as causing a departure from the scope of the claims.

The hardware used to implement various illustrative logics, logical blocks, modules, and circuits described in connection with the aspects disclosed herein may be implemented or performed with a general purpose processor, a digital signal processor (DSP), an ASIC, a field programmable gate array (FPGA) or other programmable logic device, discrete gate or transistor logic, discrete hardware components, or any combination thereof designed to perform the functions described herein. A general-purpose processor may be a microprocessor, but, in the alternative, the processor may be any conventional processor, controller, microcontroller, or state machine. A processor may also be implemented as a combination of receiver smart objects, e.g., a combination of a DSP and a microprocessor, a plurality of microprocessors, one or more microprocessors in conjunction with a DSP core, or any other such configuration. Alternatively, some operations or methods may be performed by circuitry that is specific to a given function.

In one or more aspects, the functions described may be implemented in hardware, software, firmware, or any combination thereof. If implemented in software, the functions may be stored as one or more instructions or code on a non-transitory computer-readable storage medium or non-transitory processor-readable storage medium. The operations of a method or algorithm disclosed herein may be embodied in a processor-executable software module or processor-executable instructions, which may reside on a non-transitory computer-readable or processor-readable storage medium. Non-transitory computer-readable or processor-readable storage media may be any storage media that may be accessed by a computer or a processor. By way of example but not limitation, such non-transitory computer-readable or processor-readable storage media may include RAM, ROM, EEPROM, FLASH memory, CD-ROM or other optical disk storage, magnetic disk storage or other magnetic storage smart objects, or any other medium that may be used to store desired program code in the form of instructions or data structures and that may be accessed by a computer. Disk and disc, as used herein, includes compact disc (CD), laser disc, optical disc, digital versatile disc (DVD), floppy disk, and Blu-ray disc where disks usually reproduce data magnetically, while discs reproduce data optically with lasers. Combinations of the above are also included within the scope of non-transitory computer-readable and processor-readable media. Additionally, the operations of a method or algorithm may reside as one or any combination or set of codes and/or instructions on a non-transitory processor-readable storage medium and/or computer-readable storage medium, which may be incorporated into a computer program product.

The preceding description of the disclosed aspects is provided to enable any person skilled in the art to make or use the claims. Various modifications to these aspects will be readily apparent to those skilled in the art, and the generic principles defined herein may be applied to other aspects without departing from the scope of the claims. Thus, the present disclosure is not intended to be limited to the aspects shown herein but is to be accorded the widest scope consistent with the following claims and the principles and novel features disclosed herein.

## Claims

1. A method executed by a processor (330) of an electronic device (310) for tracking hydration of a user, comprising:
receiving (440) an indication of a type of liquid consumed by the user,
determining (420) an amount of liquid (25) consumed by the user (5) based on a predetermined baseline consumption rate for the user and a number of at least one of sips or swallows by the user detected by a sensor coupled to the processor;
determining a hydration level of the user based on the determined amount of liquid consumed and further based on the type of liquid consumed by the user;
reporting (422) an indication of the determined hydration level of the user.

2. The method of claim 1, further comprising:
determining (424) the predetermined baseline consumption rate for the user based on a previously counted number of at least one of sips or swallows detected by the sensor (230) when the user consumed a known amount of liquid.

3. The method of claim 1, further comprising:
determining (426) the predetermined baseline consumption rate for the user based on a manual user input indicating at least one of a known amount of liquid or a number of sips or swallows it took to consume the known amount of liquid.

4. The method of claim 1, further comprising:
determining (428) the predetermined baseline consumption rate for the user based on a context in which the liquid was consumed.

5. The method of claim 1, further comprising:
determining a type of liquid being consumed; and
selecting (430) the predetermined baseline consumption rate for the user based on the determined type of liquid being consumed.

6. The method of claim 1, further comprising:
determining (432) an update to a previously determined baseline consumption rate for the user based on liquid consumption information from at least one of the sensor, a user input, or a context in which the liquid was consumed,
wherein the predetermined baseline consumption rate is based on the determined update to the previously determined baseline consumption rate for the user.

7. The method of claim 1, further comprising:
receiving (434) contextual information indicating a context in which the number of at least one of sips or swallows were performed by the user; and
determining (436) the hydration level of the user further based in part on the context in which the number of at least one of sips or swallows were performed by the user.

8. The method of claim 1, further comprising:
determining (438) whether the user is consuming solids in combination with the amount of liquid consumed, wherein determining the amount of liquid consumed by the user is based on the number of sips by the user in response to determining that the user is consuming solids in combination with liquid.

9. The method of claim 1, wherein the received indication of the type of liquid consumed by the user indicates that the liquid consumed by the user includes at least one of a diuretic or dehydrating substance (545), wherein determining the hydration level of the user based on the determined amount of liquid consumed comprises adjusting the hydration level lower due to the diuretic or dehydrating substance indicated in the received indication.

10. The method of claim 1, wherein the received indication of the type of liquid consumed by the user indicates that the liquid consumed by the user includes an anti-diuretic (545), wherein determining the hydration level of the user based on the determined amount of liquid consumed comprises adjusting the hydration level higher if no anti-diuretic was indicated.

11. The method of claim 1, further comprising:
receiving (442) food consumption information associated with food consumed by the user; and
determining (444) the hydration level of the user further based in part on the received food consumption information.

12. An electronic device (310), comprising:
memory; and
a processor coupled to the memory and configured with processor-executable instructions to:
receive (440) an indication of a type of liquid consumed by the user,
determine (420) an amount of liquid (25) consumed by a user (5) based on a predetermined baseline consumption rate for the user and a number of at least one of sips or swallows by the user detected by a sensor coupled to the processor;
determine a hydration level of the user based on the determined amount of liquid consumed and further based on the type of liquid consumed by the user;
report (422) an indication of the determined hydration level of the user.

13. The electronic device of claim 13, wherein the processor is further configured with processor-executable instructions to perform the method of any of claims 2 to 11.

14. A non-transitory, processor-readable medium having stored thereon processor-executable instructions configured to cause the processor of the electronic device of claim 12 to perform the method of any of claims 1 to 11.

## Patentansprüche

1. Verfahren, ausgeführt von einem Prozessor (330) eines elektronischen Geräts (310) zum Verfolgen der Hydratation eines Benutzers, das Folgendes beinhaltet:
Empfangen (440) einer Angabe eines Typs von von dem Benutzer aufgenommener Flüssigkeit,
Bestimmen (420) einer vom Benutzer (5) aufgenommenen Flüssigkeitsmenge (25) auf der Basis einer vorbestimmten Baseline-Aufnahmerate für den Benutzer und einer Anzahl von von einem mit dem Prozessor gekoppelten Sensor erkannten Trink- und/oder Schluckbewegungen des Benutzers;
Bestimmen eines Hydratationsniveaus des Benutzers auf der Basis der bestimmten Menge an aufgenommener Flüssigkeit und ferner auf der Basis des Typs von vom Benutzer aufgenommener Flüssigkeit;
Melden (422) einer Angabe des bestimmten Hydratationsniveaus des Benutzers.

2. Verfahren nach Anspruch 1, das ferner Folgendes beinhaltet:
Bestimmen (424) der vorbestimmten Baseline-Aufnahmerate für den Benutzer auf der Basis einer zuvor gezählten Anzahl von Trink- und/oder Schluckbewegungen, die vom Sensor (230) erkannt wurden, als der Benutzer eine bekannte Flüssigkeitsmenge aufnahm.

3. Verfahren nach Anspruch 1, das ferner Folgendes beinhaltet:
Bestimmen (426) der vorbestimmten Baseline-Aufnahmerate für den Benutzer auf der Basis einer manuellen Benutzereingabe, die eine bekannte Flüssigkeitsmenge und/oder eine zum Aufnehmen der bekannten Flüssigkeitsmenge erforderliche Anzahl von Trink- oder Schluckbewegungen angibt.

4. Verfahren nach Anspruch 1, das ferner Folgendes beinhaltet:
Bestimmen (428) der vorbestimmten Baseline-Aufnahmerate für den Benutzer auf der Basis eines Kontexts, in dem die Flüssigkeit aufgenommen wurde.

5. Verfahren nach Anspruch 1, das ferner Folgendes beinhaltet:
Bestimmen eines Typs von aufgenommener Flüssigkeit; und
Auswählen (430) der vorbestimmten Baseline-Aufnahmerate für den Benutzer auf der Basis des bestimmten Typs von aufgenommener Flüssigkeit.

6. Verfahren nach Anspruch 1, das ferner Folgendes beinhaltet:
Bestimmen (432) einer Aktualisierung einer zuvor bestimmten Baseline-Aufnahmerate für den Benutzer auf der Basis von Flüssigkeitsaufnahmeinformationen von mindestens einem von dem Sensor, einer Benutzereingabe und einem Kontext, in dem die Flüssigkeit aufgenommen wurde,
wobei die vorbestimmte Baseline-Aufnahmerate auf der bestimmten Aktualisierung der zuvor bestimmten Baseline-Aufnahmerate für den Benutzer basiert.

7. Verfahren nach Anspruch 1, das ferner Folgendes beinhaltet:
Empfangen (434) von Kontextinformationen, die einen Kontext angeben, in dem die Anzahl der Trink- und/oder Schluckbewegungen vom Benutzer ausgeführt wurde; und
Bestimmen (436) des Hydratationsniveaus des Benutzers ferner teilweise auf der Basis des Kontexts, in dem die Anzahl von Trink- und/oder Schluckbewegungen vom Benutzer ausgeführt wurde.

8. Verfahren nach Anspruch 1, das ferner Folgendes beinhaltet:
Feststellen (438), ob der Benutzer feste Nahrung in Kombination mit der aufgenommenen Flüssigkeitsmenge aufnimmt, wobei das Bestimmen der vom Benutzer aufgenommenen Flüssigkeitsmenge auf der Anzahl von Trinkbewegungen des Benutzers basiert, die als Reaktion auf die Feststellung erfolgt, dass der Benutzer feste Nahrung in Kombination mit Flüssigkeit aufnimmt.

9. Verfahren nach Anspruch 1, wobei die empfangene Angabe des Typs von vom Benutzer aufgenommener Flüssigkeit angibt, dass die vom Benutzer aufgenommene Flüssigkeit eine diuretische und/oder dehydrierende Substanz (545) enthält, wobei das Bestimmen des Hydratationsniveaus des Benutzers auf der Basis der bestimmten Menge an aufgenommener Flüssigkeit das Herunterjustieren des Hydratationsniveaus aufgrund der in der empfangenen Angabe angegebenen diuretischen oder dehydrierenden Substanz beinhaltet.

10. Verfahren nach Anspruch 1, wobei die empfangene Angabe des Typs von vom Benutzer aufgenommener Flüssigkeit angibt, dass die vom Benutzer aufgenommene Flüssigkeit ein Antidiuretikum (545) enthält, wobei das Bestimmen des Hydratationsniveaus des Benutzers auf der Basis der bestimmten Menge an aufgenommener Flüssigkeit das Heraufjustieren des Hydratationsniveaus beinhaltet, wenn kein Antidiuretikum angegeben wurde.

11. Verfahren nach Anspruch 1, das ferner Folgendes beinhaltet:
Empfangen (442) von Lebensmittelaufnahmeinformationen, die mit vom Benutzer aufgenommener Nahrung assoziiert sind; und
Bestimmen (444) des Hydratationsniveaus des Benutzers ferner teilweise auf der Basis der empfangenen Lebensmittelaufnahmeinformationen.

12. Elektronisches Gerät (310), das Folgendes umfasst:
einen Speicher; und
einen mit dem Speicher gekoppelten Prozessor, der mit prozessorausführbaren Befehlen konfiguriert ist zum:
Empfangen (440) einer Angabe eines Typs von von dem Benutzer aufgenommener Flüssigkeit,
Bestimmen (420) einer vom Benutzer (5) aufgenommenen Flüssigkeitsmenge (25) auf der Basis einer vorbestimmten Baseline-Aufnahmerate für den Benutzer und einer Anzahl von von einem mit dem Prozessor gekoppelten Sensor erkannten Trink- und/oder Schluckbewegungen des Benutzers;
Bestimmen eines Hydratationsniveaus des Benutzers auf der Basis der bestimmten Menge an aufgenommener Flüssigkeit und ferner auf der Basis des Typs von vom Benutzer aufgenommener Flüssigkeit;
Melden (422) einer Angabe des bestimmten Hydratationsniveaus des Benutzers.

13. Elektronisches Gerät nach Anspruch 13, wobei der Prozessor ferner mit prozessorausführbaren Befehlen zum Durchführen des Verfahrens nach einem der Ansprüche 2 bis 11 konfiguriert ist.

14. Nichtflüchtiges, prozessorlesbares Medium, auf dem prozessorausführbare Befehle gespeichert sind, die zum Veranlassen des Prozessors des elektronischen Geräts nach Anspruch 12 zum Durchführen des Verfahrens nach einem der Ansprüche 1 bis 11 konfiguriert sind.

## Revendications

1. Procédé exécuté par un processeur (330) d'un dispositif électronique (310) pour le suivi de l'hydratation d'un utilisateur, comprenant :
la réception (440) d'une indication d'un type de liquide consommé par l'utilisateur,
la détermination (420) d'une quantité de liquide (25) consommée par l'utilisateur (5) sur la base d'un taux de consommation de référence prédéterminé pour l'utilisateur et d'un nombre d'au moins une gorgée ou déglutition par l'utilisateur détectée par un capteur couplé au processeur ;
la détermination d'un niveau d'hydratation de l'utilisateur sur la base de la quantité de liquide consommée déterminée et sur la base en outre du type de liquide consommé par l'utilisateur ;
la signalisation (422) d'une indication du niveau d'hydratation déterminé de l'utilisateur.

2. Procédé selon la revendication 1, comprenant en outre :
la détermination (424) du taux de consommation de référence prédéterminé pour l'utilisateur sur la base d'un nombre précédemment compté d'au moins une gorgée ou déglutition détectée par le capteur (230) lorsque l'utilisateur a consommé une quantité connue de liquide.

3. Procédé selon la revendication 1, comprenant en outre :
la détermination (426) du taux de consommation de référence prédéterminé pour l'utilisateur sur la base d'une entrée d'utilisateur manuelle indiquant au moins une quantité connue de liquide ou un nombre de gorgées ou de déglutitions qu'il a fallu pour consommer la quantité connue de liquide.

4. Procédé selon la revendication 1, comprenant en outre :
la détermination (428) du taux de consommation de référence prédéterminé pour l'utilisateur sur la base d'un contexte dans lequel le liquide a été consommé.

5. Procédé selon la revendication 1, comprenant en outre :
la détermination d'un type de liquide consommé ; et
la sélection (430) du taux de consommation de référence prédéterminé pour l'utilisateur sur la base du type de liquide consommé déterminé.

6. Procédé selon la revendication 1, comprenant en outre :
la détermination (432) d'une mise à jour d'un taux de consommation de référence précédemment déterminé pour l'utilisateur sur la base d'informations de consommation de liquide provenant d'au moins un capteur, une entrée d'utilisateur ou un contexte dans lequel le liquide a été consommé,
dans lequel le taux de consommation de référence prédéterminé est basé sur la mise à jour déterminée du taux de consommation de référence précédemment déterminé pour l'utilisateur.

7. Procédé selon la revendication 1, comprenant en outre :
la réception (434) d'informations contextuelles indiquant un contexte dans lequel le nombre d'au moins une gorgée ou déglutition a été réalisé par l'utilisateur ; et
la détermination (436) du niveau d'hydratation de l'utilisateur sur la base en partie du contexte dans lequel le nombre d'au moins une gorgée ou déglutition a été réalisé par l'utilisateur.

8. Procédé selon la revendication 1, comprenant en outre :
la détermination (438) que l'utilisateur consomme ou non des solides en combinaison avec la quantité de liquide consommée, dans lequel la détermination de la quantité de liquide consommée par l'utilisateur est basée sur le nombre de gorgées par l'utilisateur en réponse à la détermination que l'utilisateur consomme des solides en combinaison avec un liquide.

9. Procédé selon la revendication 1, dans lequel l'indication reçue du type de liquide consommé par l'utilisateur indique que le liquide consommé par l'utilisateur comporte au moins une substance diurétique ou déshydratante (545), dans lequel la détermination du niveau d'hydratation de l'utilisateur sur la base de la quantité de liquide consommée déterminée comprend l'ajustement du niveau d'hydratation à la baisse en raison de la substance diurétique ou déshydratante indiquée dans l'indication reçue.

10. Procédé selon la revendication 1, dans lequel l'indication reçue du type de liquide consommé par l'utilisateur indique que le liquide consommé par l'utilisateur comporte un antidiurétique (545), dans lequel la détermination du niveau d'hydratation de l'utilisateur sur la base de la quantité de liquide consommée déterminée comprend un ajustement du niveau d'hydratation à la hausse si aucun antidiurétique n'a été indiqué.

11. Procédé selon la revendication 1, comprenant en outre :
la réception (442) d'informations de consommation alimentaire associée aux aliments consommés par l'utilisateur ; et
la détermination (444) du niveau d'hydratation de l'utilisateur en outre sur la base en partie des informations de consommation alimentaire reçues.

12. Dispositif électronique (310), comprenant :
une mémoire ; et
un processeur couplé à la mémoire et configuré avec des instructions exécutables par le processeur pour :
recevoir (440) une indication d'un type de liquide consommé par l'utilisateur,
déterminer (420) une quantité de liquide (25) consommée par un utilisateur (5) sur la base d'un taux de consommation de référence prédéterminé pour l'utilisateur et d'un nombre d'au moins une gorgée ou déglutition par l'utilisateur détectée par un capteur couplé au processeur ;
déterminer le niveau d'hydratation de l'utilisateur sur la base de la quantité de liquide consommée déterminée et sur la base du type de liquide consommé par l'utilisateur ;
signaler (422) une indication du niveau d'hydratation déterminé de l'utilisateur.

13. Dispositif électronique selon la revendication 13, dans lequel le processeur est configuré en outre avec des instructions exécutables par processeur pour réaliser le procédé selon l'une quelconque des revendications 2 à 11.

14. Support non transitoire, lisible par le processeur, sur lequel sont stockées des instructions exécutables par processeur configurées pour amener le processeur du dispositif électronique selon la revendication 12 à réaliser le procédé selon l'une quelconque des revendications 1 à 11.
